(19) Europäisches Patentamt / European Patent Office / Office européen des brevets

(11) **EP 4 644 415 A1**

(12) **EUROPEAN PATENT APPLICATION**

(43) Date of publication:
**05.11.2025** Bulletin 2025/45

(21) Application number: **24173920.0**

(22) Date of filing: **02.05.2024**

(51) International Patent Classification (IPC):
*C07K 16/18* $^{(2006.01)}$    *A61P 7/02* $^{(2006.01)}$

(52) Cooperative Patent Classification (CPC):
**C07K 16/18; A61P 7/02; C12N 9/6424;**
C07K 2317/22; C07K 2317/569; C07K 2317/76;
C07K 2319/33

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Synapse B.V.**
**6217 KD Maastricht (NL)**

(72) Inventor: **The designation of the inventor has not yet been filed**

(74) Representative: **Brantsandpatents bv
Pauline Van Pottelsberghelaan 24
9051 Ghent (BE)**

Remarks:
The complete document including Reference Table(s) and the Sequence Listing(s) can be downloaded from the EPO website

(54) **ANTI-FIBRIN AGENT**

(57) The current invention relates to a polypeptide comprising an immunoglobulin single variable domain and an urokinase-type plasminogen activator (uPA) domain wherein the immunoglobulin single variable domain is able to bind to fibrin via a fibrin-binding domain.

FIG. 1

**Description**

**FIELD OF THE INVENTION**

The field of the invention pertains to biochemistry and medicine, specifically to the development of novel polypeptides for therapeutic applications.

**BACKGROUND**

Hemostasis is a physiological process that allows rapid, localized, and highly regulated closure of an injured blood vessel while maintaining normal blood flow. It involves platelets (primary hemostasis), coagulation factors (secondary hemostasis), as well as components of the vessel wall. During coagulation, substantial amounts of thrombin are generated on procoagulant membranes by factor Xa-induced cleavage of plasma prothrombin. Thrombin generation is triggered by the extrinsic (via tissue factor) or intrinsic (via factor XII) coagulation pathways. Thrombin, as a crucial proteolytic enzyme, has multiple functional substrates, including coagulation factors (V, VIII, XI), the protease-activated receptors (PAR) on platelets and endothelial cells and fibrinogen, which is cleaved into fibrin monomers. The formed thrombin is inactivated by binding to antithrombin and other serpins. On the other hand, thrombin suppresses its own formation by interacting with endothelial-expressed thrombomodulin, which by cleavage generates the anticoagulant, activated protein C, thus providing spatial heterogeneity of thrombin's activity.

Thrombin generation assays involve the activation of plasma or whole blood samples using tissue factor or factor XII to monitor subsequent formation and inactivation of thrombin over a period of time. Current thrombin generation assays are capable to estimate the procoagulant and anticoagulant contributions to the clotting process, and are sensitive for conditions of hemophilia as well as thrombophilia. However, their clinical significance is only currently systematically surveyed. In high-throughput mode, thrombin generation assays use patient (platelet-rich) plasma or whole blood samples with a low-affinity fluorogenic substrate, which is cleaved by the generated thrombin so that interference with the coagulation process is minimal. It is assumed that all the formed thrombin contributes to the coagulation process in a similar manner.

One major (patho)physiological role of thrombin is the cleavage of fibrinogen molecules. Fibrinogen is a heterotrimeric molecule of A$\alpha$, B$\beta$ and $\gamma$ chains, from which thrombin N-terminally cleaves the fibrinopeptides A and B (designated as FPA and FPB). This results in so-called fibrin monomers, which then polymerize via half-staggered oligomers to protofibrils (10-50 monomers) and subsequently to growing and thickening fibrin fibers. The ultimate result is an insoluble fibrin fiber matrix, which provides structure and stability to a clot and thrombus, such as formed in hemostasis and arterial or venous thrombosis.

After cleaving off the fibrinopeptides, thrombin remains bound to the fibrin nodules near the polymerization sites. In 2004, a crystal structure of the thrombin-fibrin complex allocated two thrombin molecules at the central E-domain of a fibrin nodule, where it is bound through its non-catalytic exosites I and II. This thrombin-fibrin binding explains in vivo observations of thrombotic venules and arterioles in mouse showing complete co-localization of labeled (pro)thrombin with a labeled fibrin clot.

Fibrin monomers, also known as antithrombin I, are known to protect added thrombin from its inactivation by antithrombin III. This property would suggest that the plasma fibrinogen level is a major determinant in the extent of thrombin generation. However, this is usually not observed. For instance, fibrinogen infusion did not alter the thrombin generation of plasma from patients with afibrinogenemia. Furthermore, the thrombin-binding splice variant g' of the fibrinogen g-chain, rather than the fibrinogen level itself, determined the thrombin generation potential in patient samples, and the polymerization of fibrin appeared to determine thrombin generation via VWF. Yet, the fibrinogen level is an important factor in haemostasis, given the frequent bleeding in patients with congenital afibrinogenemia or dysfibrinogenemia and the need for fibrinogen infusion in patients with perioperative dilutional coagulopathy.

Acute thrombosis is one of the leading causes of mortality in developed societies and is caused by fibrin clot formation. Anti-fibrin therapy, crucial in the initial hours following an acute thrombotic event, involves the enzymatic breakdown of blood clots.

Currently, the most effective agent for this purpose is recombinant tissue plasminogen activator (rtPA), which specifically activates plasminogen into plasmin on the surface of fibrin. The efficacy of rtPA is primarily due to its fibrin-binding finger domain and its catalytic domain that activates plasminogen. However, rtPA is relatively unstable when dissolved in physiological buffers, complicating treatment logistics. Additionally, the production of tPA is challenging due to its complexity, and manufacturing issues can lead to reduced availability of this crucial therapeutic agent.

There is, therefore, a need for an anti-fibrin agent that is more stable, easier to produce, and as effective, if not more so, than rtPA.

Moreover, when plasminogen activators are not specifically targeted by an antibody or other mechanisms, they can bind nonspecifically in several areas. This nonspecific activation can lead to systemic effects, such as unwanted tissue breakdown or excessive bleeding. This issue is typically addressed by linking the plasminogen activator to an antibody. However, antibodies commonly used are known to cross-react with fibrinogen.

Thus, there is a need for an antibody linked to a plasminogen activator that ensures fibrinolytic activity is directed precisely at blood clots. This would enhance the safety and effectiveness of the treatment by minimizing the risks associated with systemic fibrinolysis and uncontrolled bleeding.

In treating thrombotic events, the choice of the protein is also essential. Prior art strategies in targeting thrombosis involve the use of agents or anticoagulants that target thrombin directly, which can potentially affect multiple aspects of thrombin's role in the coagulation cascade, not just the conversion of fibrinogen to fibrin. This broad inhibition could lead to systemic effects, such as an increased risk of bleeding in areas where clotting is necessary for healing or maintenance. Additionally, fibrin-independent agents could diffuse more widely, diluting their effectiveness where it is most needed and potentially causing unwanted anticoagulation throughout the circulatory system. The body's coagulation system is highly adaptive and can sometimes develop mechanisms to bypass inhibitors. A generalized inhibitor of thrombin may prompt compensatory mechanisms that reduce its effectiveness over time or necessitate higher, riskier dosages.

There is thus a need for a thrombolytic agent with a higher specificity to the clot site and localized action.

The present invention addresses these issues by providing a novel fibrinolytic agent that includes both a fibrin-binding domain and a fibrinolytic catalytic domain. The catalytic domain utilizes a recombinant urokinase-type plasminogen activator (uPA), which is smaller, more potent, and easier to produce than tPA. The fibrin-binding domain comprises a VHH derived from a llama antibody, specifically selected for its specificity toward fibrin and its avoidance of cross-reactivity with fibrinogen. Notably, the novel uPA-anti-fibrin fusion protein can be produced in high quantities, offering a promising solution to the limitations of current treatments.

## SUMMARY OF THE INVENTION

The present invention and embodiments thereof serve to provide a solution to one or more of above-mentioned disadvantages. To this end, the present invention relates to a novel polypeptide comprising an immunoglobulin single variable domain and a urokinase-type plasminogen activator (uPA) domain. The single variable domain can bind to fibrin, making this polypeptide a potent fibrinolytic and thrombolytic agent.

The polypeptide can exhibit higher fibrinolytic activity than conventional uPAs and can have a high affinity for fibrin over fibrinogen, leading to efficient clot degradation.

The polypeptide is also described with specific amino acid sequences, offering consistency in its structural and functional characteristics.

Furthermore, the polypeptide can be formulated into a composition with a pharmaceutical carrier, excipient or diluent, and can be used for treating diseases or conditions characterized by a thrombotic or embolic state.

The polypeptide's enhanced potency and stability, along with its ability to specifically bind to fibrin and efficiently degrade clots, make it a promising candidate for acute thrombosis treatment and other thrombotic or embolic conditions.

## DESCRIPTION OF FIGURES

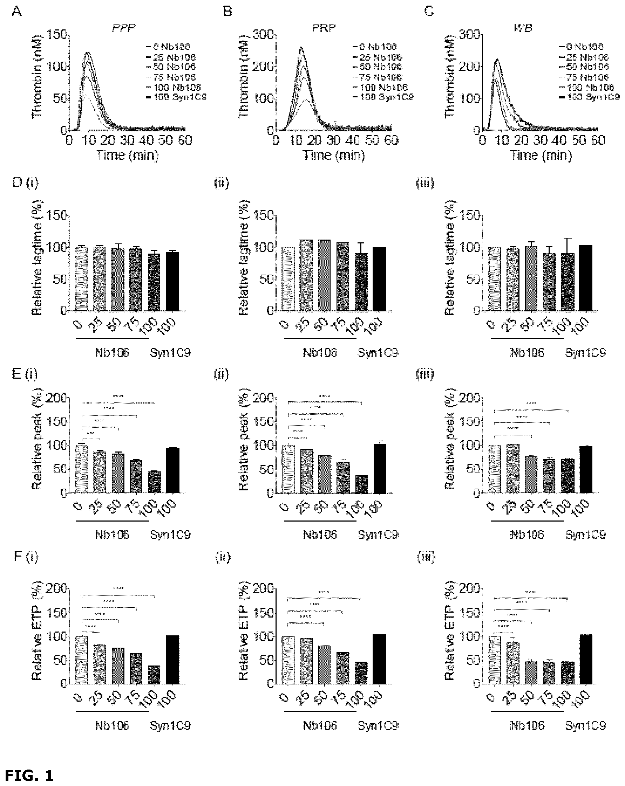

**Figure 1. Major reduction of thrombin generation in plasma and whole blood by Nb106.** Parallel samples of platelet-poor plasma (PPP), platelet-rich plasma (PRP) and whole blood (WB) were pre-incubated with vehicle control solution, Nb106 (25 to 100 $\mu$g/mL) or indifferent Nb-Syn1C9 (100 $\mu$g/mL). Thrombin generation was induced with 1 pM tissue factor in the presence of 11 mM $CaCl_2$, 5.5 mM 5.5 mM $MgCl_2$ and 4 $\mu$M phospholipids. Quantification was from the cleavage rate of Z-GGR-AMC. Shown are representative thrombin generation curves with autologous PPP **(Ai),** PRP **(Aii)** and WB **(Aiii).** Values of curve parameters, thrombin lagtime **(B),** peak height **(C)** and ETP **(D),** are given as percentages of the control condition without Nb106. Parameter values for PPP **(i),** PRP **(ii)** and whole blood **(iii).** Means $\pm$ SD (n=3 experiments). ***P < 0.005, ****p < 0.0001 (one-way ANOVA).

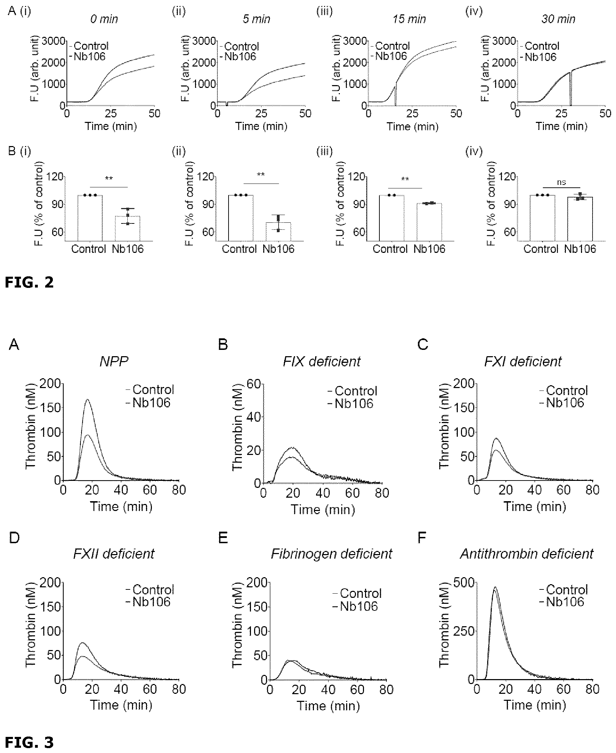

**Figure 2. Time-dependency of reduction in thrombin generation.** Normal pool plasma (NPP) was incubated with vehicle control solution or Nb106 (100 $\mu$g/mL) 0 min (i), 5 min **(ii),** 15 min **(iii)** or 30 min **(iv)** after triggering of thrombin generation with 1 pM tissue factor and phospholipids, as for Figure 1. Shown are raw curves of AMC fluorescence accumulation **(A)** and normalized fluorescence values after 50 min **(B).** Note gradual decline of effect of Nb106. Data are means $\pm$ SD (n=3 experiments), **p < 0.01 (t-test).

**Figure 3. Fibrinogen and antithrombin both required for reduced thrombin generation.** Thrombin generation in various plasma types was induced as in Figure 1. Samples were pre-incubated with vehicle control solution or Nb106 (100 $\mu$g/mL). Compared were normal pool plasma (NPP) **(A),** and plasmas deficient in factor IX **(B),** factor XI **(C),** factor XII **(D),** fibrinogen **(E),** or antithrombin **(F).** Shown are mean thrombin generation curves from triplicate experiments. Quantified curve parameters with statistics are given in panel G.

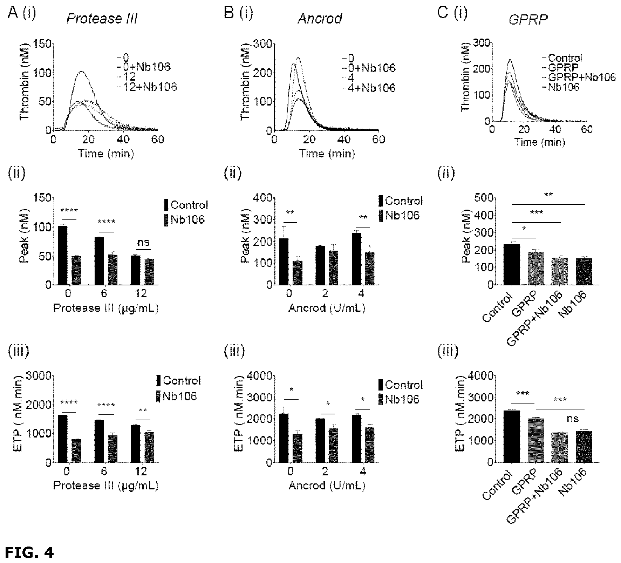

**Figure 4. Protease III cleavage abolishes reduced thrombin generation.** Thrombin generation in normal pool plasma was induced with vehicle control solution or Nb106 (100 $\mu$g/mL), as in Figure 1. To alter fibrin formation, plasma samples were preincubated with 12 μg/mL protease III **(A),** 4 U/mL ancrod **(B)** or 4 mM GPRP **(C).** Shown are representative thrombin generation curves **(i),** thrombin peak heights **(ii),** and ETP values **(iii).** Means ± SD (n=3 experiments). *P < 0.05, **p < 0.01, ***p < 0.005, ****p < 0.0001 (two- or one-way ANOVA).

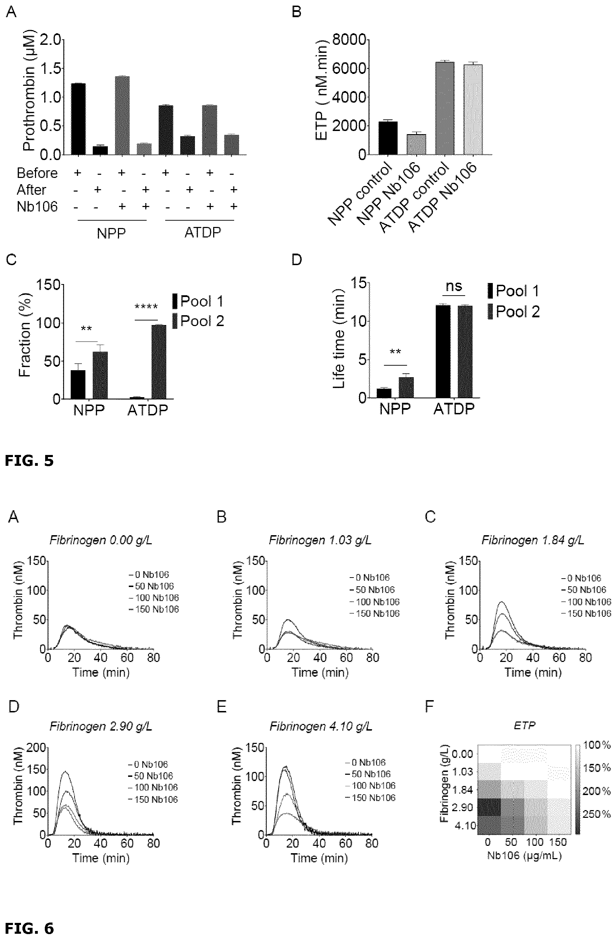

**Figure 5. Fibrin-independent and -dependent thrombin pools 1 and 2.** Normal pool plasma (NPP) and antithrombin-deficient plasma (ATDP) were activated in the presence or absence of Nb106 (100 μg/mL), as in Figure 1. Shown are prothrombin levels before and after activation **(A),** and furthermore measured ETP values **(B).** Based on the ETP reduction with Nb106, calculated were fractions of pool 1 (Nb106-insensitive, fibrin-independent in NPP), and pool 2 (Nb106-sensitive, fibrin-dependent in NPP) **(C).** Estimated life times of thrombin pools 1 and 2 **(D),** based on prothrombin consumption and ETP values. Data are means ± SD (n= 3 experiments), **p < 0.01, ****p < 0.0001 (two-way ANOVA).

**Figure 6. Effect of Nb106 is dependent on fibrinogen concentration.** Fibrinogen-deficient plasma was reconstituted with purified human fibrinogen (0-4.10 g/L, final concentrations), and pre-incubated with various doses of Nb106 (0, 50, 100 or 150 μg/mL). Thrombin generation was triggered with tissue factor/phospholipids, as in Figure 1. Shown are representative curves at indicated fibrinogen concentrations **(A-E).** Furthermore, a matrix heat chart of ETP values (nM x min) is shown **(F).** Means ± SD (n= 3).

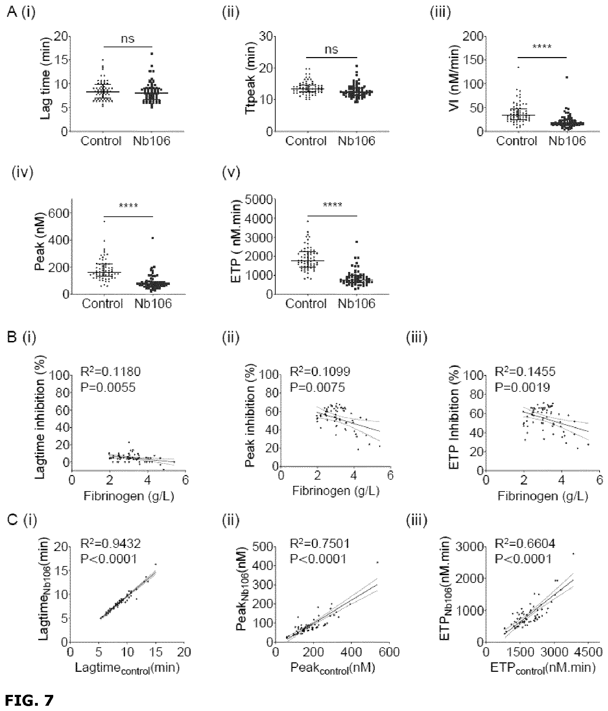

**Figure 7. Variability of fibrin-dependent thrombin generation in healthy subjects.** Plasmas from 64 healthy subjects were pre-incubated with vehicle solution or Nb106 (150 μg/mL). Thrombin generation was then induced with tissue factor (1 pM), as for Figure 1. **A,** Indicated are per subject curve parameter values without or with nanobody, concerning thrombin lag time **(i),** time-to-peak **(ii),** velocity index **(iii),** peak level **(iv)** and ETP **(v). B,** Scatter plots of percentage inhibition per subject as function of the plasma fibrinogen level for thrombin lagtime **(i),** peak height **(ii)** and ETP **(iii). C,** Scatter plots of curve parameters with or without Nb106 for thrombin lagtime **(i),** peak height **(ii)** and ETP **(iii).** N=64 subjects, ****p < 0.0001 (Mann-Whitney t-test). The coefficient of determination $R^2$ was also as indicated.

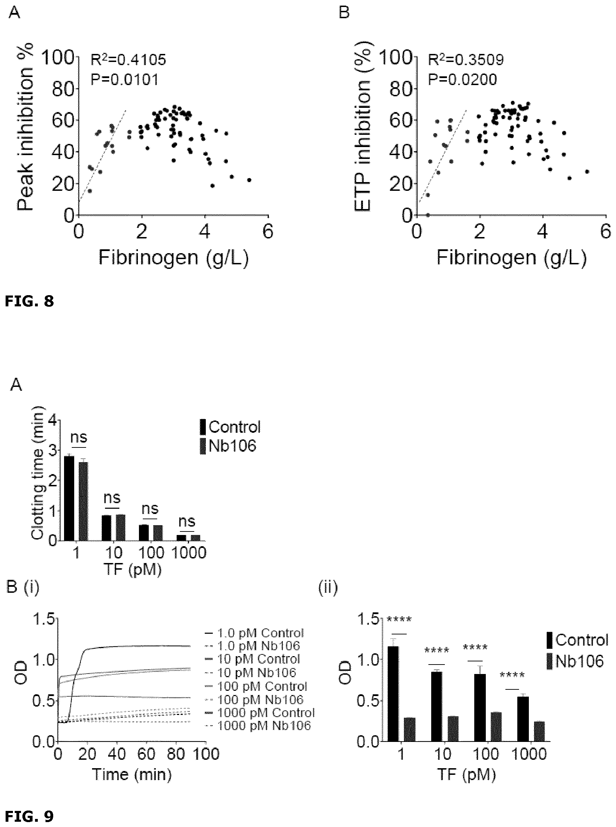

**Figure 8. Fibrin-dependent thrombin generation in patients with congenital dysfibrinogenemia.** Plasma samples from patients (Table 1) and healthy subjects (Figure 7) were incubated with vehicle control solution or Nb106 (150 μg/mL) and triggered for thrombin generation with 1 pM tissue factor, as in Figure 1. Dot plots of thrombin peak height **(A)** and ETP **(B)** percentual inhibition and functional fibrinogen level (Clauss). The coefficient of determination $R^2$ in patients' samples was also as indicated.

**Figure 9. Contribution of fibrin-bound thrombin to plasma clotting time and plasma turbidity change.** Plasmas from healthy subjects were triggered with 1-1000 pM tissue factor (TF) and phospholipids, as in Figure 1. Preincubation was with vehicle solution or Nb106 (100 μg/mL), replacing fibrin-bound thrombin. **A,** Effect on TF-induced mechanical plasma clotting time. **B,** TF-induced turbidity change over time (i) and optical density after 80 min **(ii).** Means ± SD, ****p < 0.0001 (n=3 or 4 experiments, two-way ANOVA).

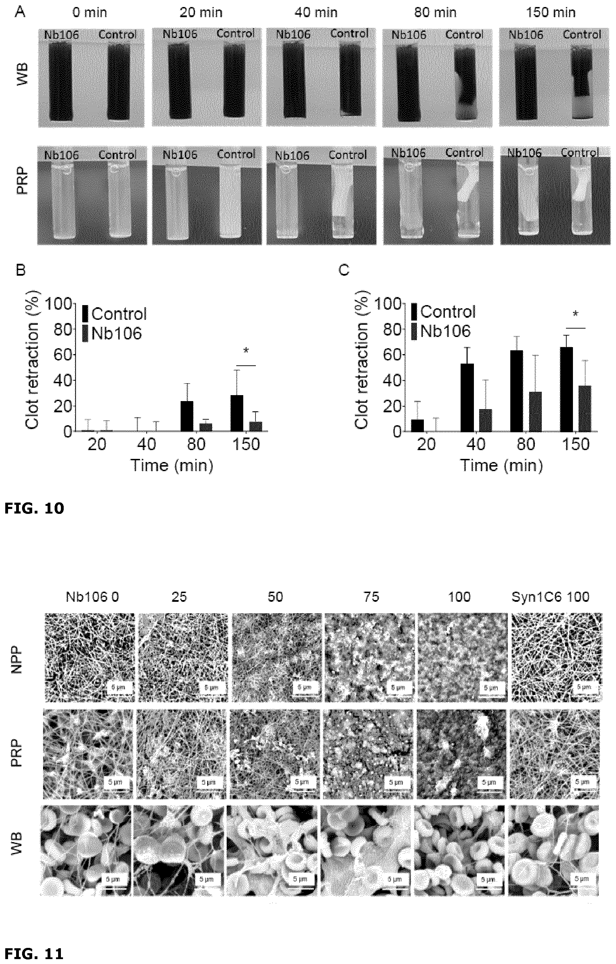

**Figure 10. Contribution of fibrin-bound thrombin to clot formation and retraction.** Coagulation was induced in autologous whole blood and PRP by recalcification (10 mM $CaCl_2$) and 1 pM tissue factor. Samples contained vehicle medium or Nb106 (100 μg/mL). **A,** For representative samples, formation and retraction of fibrin clot was imaged after 0, 20, 40, 80 and 150 min. **B,** Quantitative image analysis of clot retraction in whole blood. **C,** Quantitative data of clot retraction in PRP. Means ± SD (n=3 or 4 experiments), *p < 0.05, ****p < 0.0001 (two-way ANOVA).

**Figure 11. Requirement of fibrin-bound thrombin for fibrin fiber extension.** Samples of autologous whole blood and PRP as well as normal pool plasma (NPP) were activated with 1 pM tissue factor, 11 mM $CaCl_2$ and 5.5 mM $MgCl_2$ for 100 min, compare Figure 9. Samples were preincubated with different concentrations of Nb106 (0-100 μg/mL) or with control nanobody Nb-Syn1C6 (100 μg/mL). Scanning electron microscopy images were prepared for visualization of the fibrin structures formed. Representative for 3 experiments.

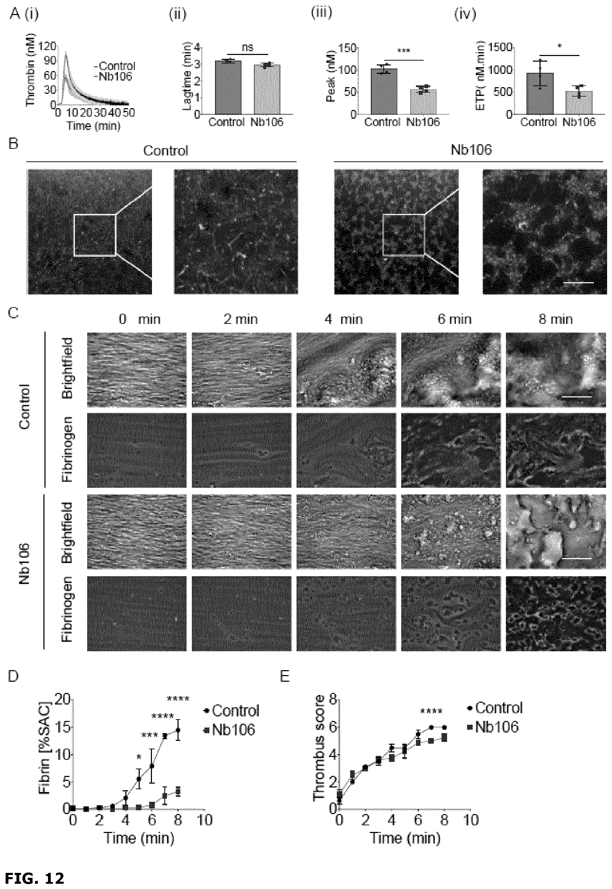

**Figure 12. Altered fibrin formation and thrombus formation ex *vivo* in mouse. A,** PRP from adult C57BL/6 mice was incubated with vehicle solution or Nb106 (100 μg/mL), and thrombin generation was induced with tissue factor (1 pM), CRP (10 μg/mL) and $CaCl_2$ (16.6 mM). Shown are representative thrombin generation curves (i), and assessed curve parameters lagtime **(ii),** peak height **(iii)** and ETP **(iv).** Mean ± SD (n = 4), *p < 0.05, ***p < 0.0005 (t-test). **B,** C57BL/6 plasma containing AF488-fibrinogen with(out) Nb106 (100 μg/mL) was triggered with 1 pM tissue factor, 11 mM $CaCl_2$ and 5.5 mM $MgCl_2$. Representative confocal fluorescence images of fibrin formation with Nb106 after 100 min. Bar = 100 mm. **C-E,** Mouse blood was supplemented with AF488-fibrinogen and AF647-anti-GPIX mAb, with Nb106 (100 μg/mL) present as indicated. Blood samples were under continuous recalcification plus tissue factor perfused over collagen-I microspots at low shear rate of 200 $s^{-1}$. **C,** Representative brightfield and fluorescence images of platelet-fibrin thrombi during flow. **D,** Quantification of fibrin surface area coverage over time. **E,** Quantification of thrombus score. Mean ± SEM (n = 4), *p < 0.05, ***p < 0.0005, ****p < 0.0001 (two-way ANOVA).

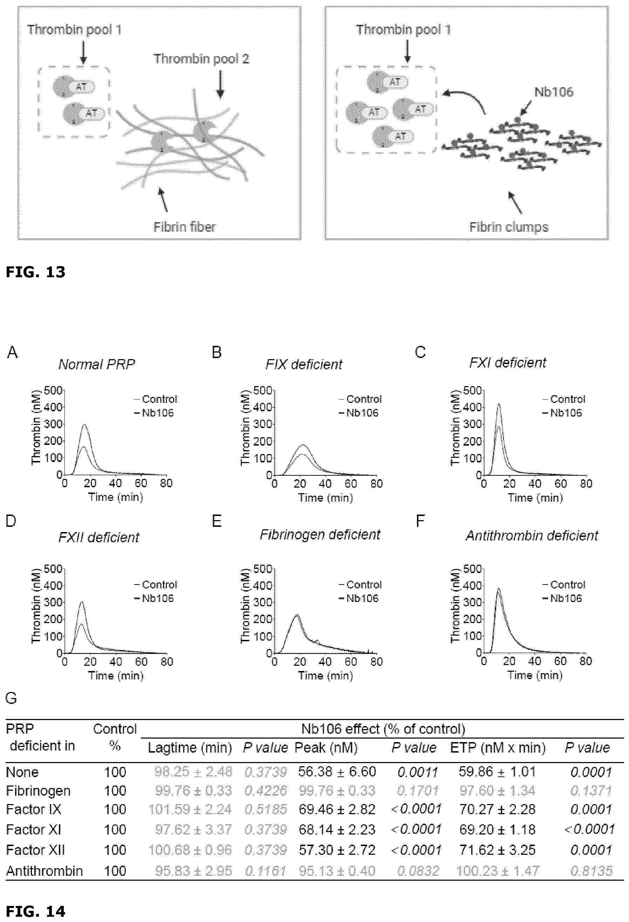

**Figure 13. Distinct functions of thrombin pools.** Under control coagulant conditions proteolytically active thrombin is divided over a fibrin-independent (pool 1) and a fibrin-dependent (pool 2) pool. Nb106 replaces the latter, eliminating its protection against antithrombin inactivation and disturbing the extension of fibrin fibers, thus resulting in fibrin clumps.

**Figure 14. Fibrinogen and antithrombin both required for reduced thrombin generation in the presence of platelets.** Normal pool plasma (NPP) or indicated factor-deficient plasmas were supplemented with platelets (2 ×

$10^8$/mL) from three donors to obtain reconstituted PRP samples. Added was also vehicle control solution or Nb106 (100 $\mu$g/mL). Calibrated thrombin generation was measured upon triggering with 1 pM tissue factor, 11 mM CaCl$_2$, 5.5 mM MgCl$_2$ and 4 $\mu$M phospholipids. Shown are representative curves with normal PRP **(A),** and PRP deficient in factor IX **(B),** factor XI **(C),** factor XII **(D),** fibrinogen **(E),** or antithrombin **(F).** Furthermore, quantified Nb106 effects on thrombin generation curve parameters **(G).** Means $\pm$ SD (n=3-5). Significant changes are in black (one-way ANOVA).

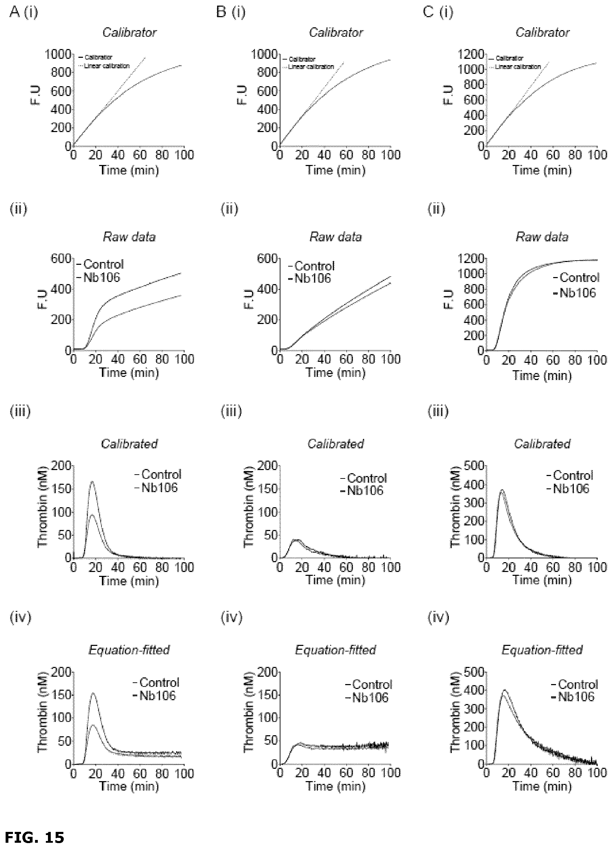

**Figure 15. Suppressed thrombin generation requires fibrinogen and antithrombin: raw curves.** Thrombin generation in various plasma types in the absence or presence of Nb106 (100 $\mu$g/mL) was induced as in Suppl. Figure 1. Used were: **(A)** normal pool plasma (NPP); **(B)** fibrinogen-deficient plasma; **(C)** antithrombin-deficient plasma. Panels **(i):** raw fluorescence traces of calibrator in indicated plasma. Dotted line = supposed linear calibrationI. Panels **(ii):** raw fluorescence traces of thrombin generation in indicated plasma (means of triplicates). Panels **(iii):** thrombin generation curves based on linear calibration curves. Panels **(iv):** thrombin generation curves obtained by comparison to calibrator wells, based on non-linear calibration until saturating fluorescence (inner filter effect and substrate depletion).

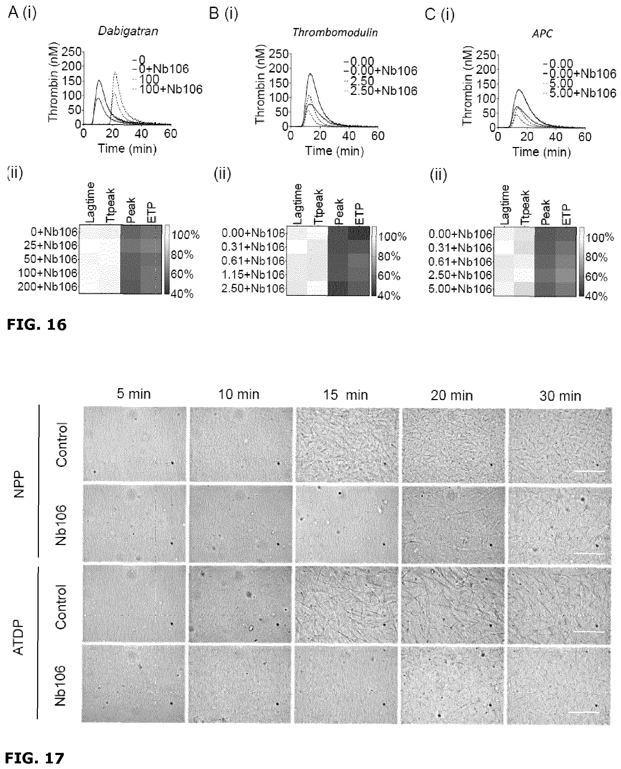

**Figure 16. Nb106 effects on thrombin generation under modulated conditions. A-C,** Plasma samples were triggered with 1 pM tissue factor, as in Suppl. Figure 1. Preincubation was with dabigatran (0-100 nM), thrombomodulin (TM, 0-2.5 nM), or activated protein C (APC, 0-5 U/L). Experiments were carried out in the absence (vehicle control) or presence of Nb106 (100 $\mu$g/mL). Shown are: (i) calibrated thrombin generation curves from a representative experiment; (ii) heatmaps indicating relative effects of Nb106 on thrombin lagtime, time-to-peak (Ttpeak), peak height, and ETP. Mean results from triplicate experiments. Color bar indicates percentage effect of Nb106 versus the corresponding control condition.

**Figure 17. Fibrin-bound thrombin required for normal fiber extension under stasis.** Transmission microscopy images of fibrin formation in plasma and antithrombin-deficient plasma in the presence or absence of Nb106. Fibrin formation in 96-well plates was triggered as in Suppl. Figure 1. Shown are representative images at 5-30 min. Bars= 100 mm.

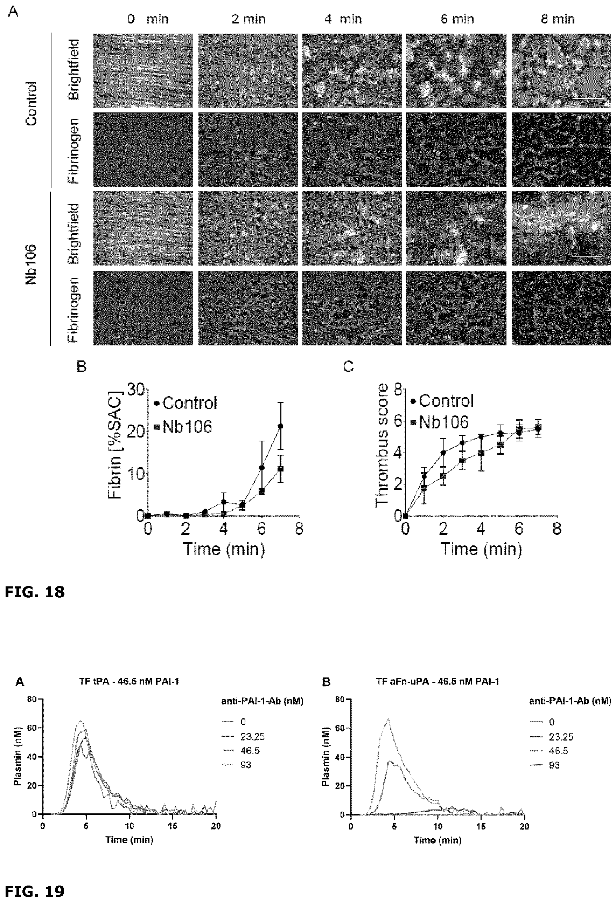

**Figure 18. Altered fibrin formation and thrombus formation ex *vivo* at high shear rate in mouse.** Mouse blood was supplemented with AF488-fibrinogen and AF647-anti-GPIX mAb, with or without Nb106 (100 $\mu$g/mL) present, as indicated. Blood samples were under continuous recalcification plus tissue factor perfused over collagen-I microspots at high shear rate of 1000 s$^{-1}$. **A,** Representative brightfield and fluorescence images of platelet-fibrin thrombi during flow. **B,** Quantification of fibrin surface area coverage over time. **C,** Quantification of thrombus score. Mean $\pm$ SEM (n = 4), *p < 0.05, ***p < 0.0005, ****p < 0.0001 (two-way ANOVA).

**Figure 19. PAI-1-induced inhibition of plasmin generation (PG) is abolished by anti-PAI-1-ab.** Normal pooled plasma, treated with 46.5 nM (2 $\mu$g/mL) PAI-1 was incubated (5 min, room temperature) with a range of anti-PAI-1-Ab concentrations, as indicated. PG induced by tissue factor (TF) and tPA **(A)** or aFn-uPA **(B),** was measured using the calibrated, automated PG assay.

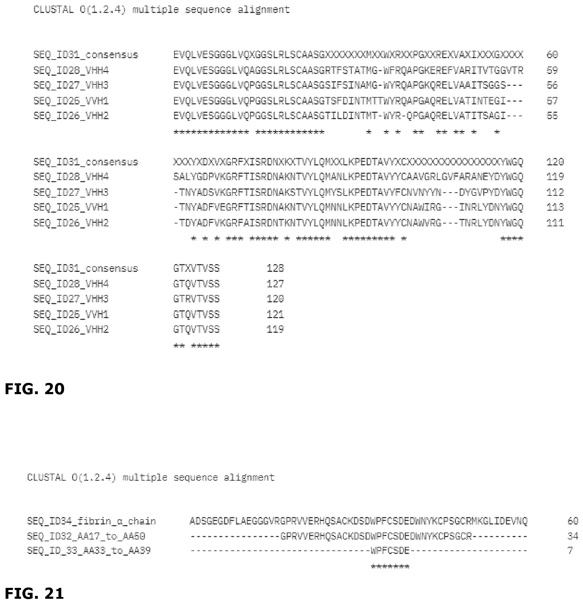

**Figure 20. Multiple Sequence Alignment of VHH Domains.** The multiple sequence alignment of variable heavy chain (VHH) domains according to some embodiment of the invention was generated by CLUSTAL O (1.2.4) software. The consensus sequence (SEQ_ID31_consensus) is shown at the top of each segment, followed by sequences from four different VHH domain variants (SEQ_ID28_VHH4, SEQ_ID27_VHH3, SEQ_ID25_VVH1, SEQ_ID26_VHH2). The alignment showcases the amino acid residues at each position, with conserved residues highlighted by asterisks below the sequences. The numbering on the right indicates the position of the last amino acid in each row relative to the full-length sequence. Gaps in the alignment are represented by dashes, indicating insertions or deletions (indels) relative to the consensus.

**Figure 21. Multiple Sequence Alignment of Fibrin $\alpha$-Chain Fragments.** The figure presents a CLUSTAL O (1.2.4) multiple sequence alignment, focusing on the initial 60 amino acids of the fibrin $\alpha$-chain (SEQ_ID34_fibrin_o_chain), aligned with specific VHH binding sites as described in an embodiment of the invention. SEQ_ID32_AA17_to_AA50 corresponds to a VHH binding site spanning amino acids 17 to 50 from the N-terminal end of the fibrin $\alpha$-chain, while SEQ_ID_33_AA33_to_AA39 delineates a narrower binding region between the 33rd and 39th amino acids. The right-hand numerical annotation indicates the amino acid positions, clarifying the relative locations of these VHH binding sites along the fibrin $\alpha$-chain.

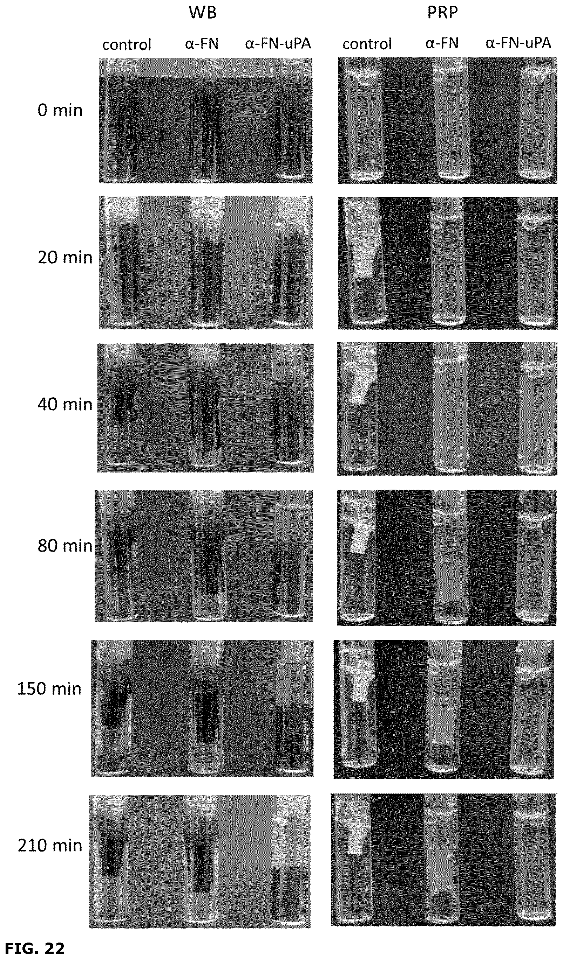

**Figure 22. Contribution of fibrin-bound thrombin to clot formation and retraction.** Coagulation was induced in autologous whole blood and PRP by recalcification (10 mM CaCl2) and 1 pM tissue factor. Samples contained vehicle medium (control), a VHH antibody according to an embodiment of the invention (o-FN) and a polypeptide according to the invention comprising VHH and uPA (o-FN-uPA). Formation and retraction of fibrin clot was imaged after 0, 20, 40, 80, 150 and 210 min.

## DETAILED DESCRIPTION OF THE INVENTION

The present invention concerns a fusion protein targeted towards treating, preventing, or ameliorating conditions characterized by thrombotic or embolic states. These include a variety of serious health conditions such as embolism, stroke, myocardial infarction, deep vein thrombosis, and other clot-related disorders.

The polypeptide as disclosed herein comprises an immunoglobulin single variable domain and a uPA domain, where the immunoglobulin domain binds fibrin at a thrombin-binding site.

## Definitions

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any $\geq 3, \geq 4, \geq 5, \geq 6$ or $\geq 7$ etc. of said members, and up to all said members.

The terms "protein" or "polypeptide" are used interchangeably and refer to molecules consisting of a chain of amino acids, without reference to a specific mode of action, size, 3-dimensional structure or origin. In amino acid sequences or protein variants as defined herein, amino acids are denoted by single-letter or three-letter symbols. These single-letter and three- letter symbols are well known to the person skilled in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gin) is glutamine, R (Arg) is arginine, S (Ser) is serine, T (Thr) is threonine, V (Val) is valine, W (Trp) is tryptophan, Y (Tyr) is tyrosine.

Unless indicated otherwise, the terms "immunoglobulin" and "antibody" whether it used herein to refer to a heavy chain antibody or to a conventional 4-chain antibody is used as a general term to include both the full-size antibody, the individual chains thereof, as well as all parts, domains or fragments thereof (including but not limited to antigen-binding domains or fragments such as VHH domains or VH/VL domains, respectively). In addition, the term "sequence" as used herein (for example in terms like "immunoglobulin sequence", "antibody sequence", "variable domain sequence", "VHH sequence" or "protein sequence"), should generally be understood to include both the relevant amino acid sequence as well as nucleic acid sequences or nucleotide sequences encoding the same, unless the context requires a more specific interpretation.

The "variable region" or "variable domain" of an antibody refers to the amino-terminal domains of the heavy or light chain of the antibody. The variable domain of the heavy chain may be referred to as "VH", or to "VHH" in case of a heavy chain antibody such as the camelid antibodies that consist of only heavy chains. The variable domain of the light chain may be referred to as "VL." These domains are generally the most variable parts of an antibody and contain the antigen-binding sites. The term "variable" refers to the fact that certain segments of the variable domains differ extensively in sequence among antibodies. The V domain mediates antigen binding and defines specificity of a particular antibody for its particular antigen. However, the variability is not evenly distributed across the average 110-amino acid span of the variable domains. Instead, the V regions consist of relatively invariant stretches called framework regions (FRs) of about 15-30 amino acids separated by shorter regions of extreme variability called "hypervariable regions" (HVRs) or complementarity determining regions (CDRs) that are each about 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies.

The terms "VHH", "VHH domain" and "nanobody" are interchangeable herein and are used herein to refer to the variable domain of a heavy chain antibody, i.e. an antibody consisting only of heavy chains and devoid of light chains as are known e.g. from Camelids. The amino acid sequence and structure of a VHH can be considered without however being limited thereto to be comprised of four framework regions or "FR's", which are referred to in the art and herein below as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDRs", which are referred to in the art as "Complementarity Determining Region 1" or "CDR1"; as "Complementarity Determining Region 2" or "CDR2"; and as "Complementarity Determining Region 3" or "CDR3", respectively. The total number of amino acid residues in a VHH can be in the region of 110-130, is preferably 119-128. It should however be noted that parts, fragments or a VHH are not particularly limited as to their length and/or size, as long as such parts, fragments or analogs meet the further functional requirements outlined herein and are also preferably suitable for the purposes described herein.

The term "urokinase-type plasminogen activator (uPA) domain" refers to a domain of the uPA enzyme that is involved in the conversion of plasminogen to plasmin, a key step in the fibrinolysis process.

The term "fibrin" refers to a fibrous protein involved in the clotting of blood. The term "fibrin-binding domain" refers to a region of a protein that can bind to fibrin.

The term "fibrinolytic activity" or "ani-fibrin activity" as interchangeably used herein, refers to the ability of a substance to break down fibrin, thereby preventing or dissolving blood clots. The term "thrombolytic activity" refers to the ability of a substance to dissolve thrombi or blood clots. The term "linker" refers to a molecule or set of molecules that connects two or more other molecules together.

Amino acid residues will be indicated according to the standard three-letter or one-letter amino acid code. For the purposes of comparing two or more nucleotide sequences, the percentage of "sequence identity" between a first nucleotide sequence and a second nucleotide sequence may be calculated or determined by dividing [the number of nucleotides in the first nucleotide sequence that are identical to the nucleotides at the corresponding positions in the second nucleotide sequence] by [the total number of nucleotides in the first nucleotide sequence] and multiplying by [100%], in which each deletion, insertion, substitution or addition of a nucleotide in the second nucleotide sequence -compared to the first nucleotide sequence - is considered as a difference at a single nucleotide (position); or using a suitable computer algorithm or technique. The degree of sequence identity between two or more nucleotide sequences may be calculated using a known computer algorithm for sequence alignment such as NCBI Blast v2.0, using standard settings. Usually, for the purpose of determining the percentage of "sequence identity" between two nucleotide sequences in accordance with the calculation method outlined hereinabove, the nucleotide sequence with the greatest number of nucleotides will be taken as the "first" nucleotide sequence, and the other nucleotide sequence will be taken as the "second" nucleotide sequence. The same calculation method applies for determining the percentage of "sequence identity" for amino acid sequences.

The term "pharmaceutical-acceptable carrier, excipient or diluent" refers to a substance that is considered safe and effective and is included in a drug to aid the delivery of the therapeutic substance.

The term "parenteral administration" refers to the administration of a substance by a route other than the digestive tract, such as intravenous, subcutaneous, or intramuscular routes. It may be any molecule which may improve the polypeptide's selectivity, effectiveness and/or safety of administration to a human or animal body, such as by continuous or triggered release or by allowing membrane permeation of the polypeptide.

The term "thrombotic or embolic state" refers to a condition characterized by the formation of blood clots within a blood vessel or the lodging of a loose clot within a blood vessel, respectively.

The term "acute or chronic disease or condition" refers to a disease or condition that has a rapid onset and/or a short course, or a disease or condition that is persistent or long-lasting, respectively

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

### Fibrin-binding polypeptides

In a first aspect, the current disclosure relates to a polypeptide comprising an immunoglobulin single variable domain and an urokinase-type plasminogen activator (uPA) domain wherein the immunoglobulin single variable domain is able to bind to fibrin via a fibrin-binding domain. This particular arrangement allows for a targeted approach to thrombolytic therapy, as the polypeptide is designed to bind specifically to fibrin, the main component of blood clots and not to fibrinogen, which is present in the blood in its soluble form and is not involved in clot formation.

The polypeptide of the invention targets fibrin directly, offering the advantage of greater specificity to clots, minimizing systemic effects and focusing the therapeutic action at the sites of thrombosis. This is particularly valuable in acute thrombotic conditions or in localized vascular territories where rapid and localized clot dissolution is necessary, such as in ischemic strokes or myocardial infarctions, without significantly impacting overall coagulation status. The specific binding to fibrin offers more predictable pharmacodynamics and pharmacokinetics, compared to thrombin-binding agents, as the activity is localized and does not diffusely affect multiple processes within the coagulation cascade. This leads to a more controlled therapeutic response. Additionally, the fibrin targeting polypeptide according to the invention ensures that the fibrinolytic activity is focused where it is most needed, on existing clots. This direct action leads to more efficient clot dissolution compared to broader thrombin inhibition, which may not necessarily enhance the breakdown of already formed clots.

The specificity of the polypeptide to selectively bind fibrin without cross-linking with fibrinogen results in the avoidance of unintended interactions with fibrinogen, which is abundant in the bloodstream and involved in the initial stages of clot formation. By circumventing fibrinogen, the polypeptide reduces the potential for triggering excessive systemic anticoagulation, which can lead to widespread bleeding disorders, a critical consideration in therapeutic applications.

Furthermore, by not binding to fibrinogen, the polypeptide preserves the normal physiological processes of clot formation and wound healing. This is particularly important in maintaining essential hemostatic balance throughout the body, ensuring that natural clotting functions proceed without disruption in response to vascular injuries or surgical interventions. Another advantage includes minimizing the risk of developing resistance or adverse immune reactions that might arise from frequent exposure to therapeutic agents that interact with fibrinogen. Such interactions can sometimes lead to the development of neutralizing antibodies or altered pharmacological profiles, complicating long-term treatment strategies.

Moreover, the lack of interaction with fibrinogen allows for a clearer therapeutic endpoint and monitoring. Clinicians can more accurately assess the effectiveness and dosing of the treatment based on its targeted action at the clot site rather than effects dispersed throughout the circulation. This can lead to more tailored and effective management of thrombotic conditions with enhanced patient outcomes.

In an embodiment of the polypeptide, as disclosed herein, the fibrin-binding domain is comprised in a single variable domain on a heavy chain (VHH) domain or a fragment thereof. The VHH domain is derived from a camelid antibody, specifically selected for its specificity toward fibrin while avoiding cross-reactivity with fibrinogen. The VHH domain is preferably "humanized" as below. The use of the single variable domain on a heavy chain or a fragment thereof enhances the stability of the polypeptide, thereby improving its shelf life and ease of storage. In addition, the single variable domain on a heavy chain also contributes to the improved binding affinity of the polypeptide to fibrin, which enhances the therapeutic effect of the polypeptide. The single variable domain on a heavy chain is easier and more cost-effective to manufacture than other types of domains, which makes the polypeptide more affordable to patients and healthcare providers.

Generally, it should be noted that the term "VHH" (or nanobody) as used herein in its broadest sense is not limited to a specific biological source or to a specific method of preparation. For example, the VHHs of the disclosure can be obtained (1) by isolating the VHH domain of a naturally occurring heavy chain antibody; (2) by expression of a nucleotide sequence encoding a naturally occurring VHH domain; (3) by "humanization" (as described below) of a naturally occurring VHH domain or by expression of a nucleic acid encoding a such humanized VHH domain; (4) by "camelization" of a naturally occurring VH domain from any animal species, in particular a species of mammal, such as from a human being, or by expression of a nucleic acid encoding such a camelized VH domain; (5) using synthetic or semisynthetic techniques for preparing proteins, polypeptides or other amino acid sequences; (6) by preparing a nucleic acid encoding a VHH using techniques for nucleic acid synthesis, followed by expression of the nucleic acid thus obtained; and/or (7) by any combination of the foregoing. Suitable methods and techniques for performing the foregoing are state of the art and

## EP 4 644 415 A1

therefore known to the skilled person.

Preferably, the VHH as disclosed herein has an amino acid sequence that corresponds to the naturally occurring VHH domain, but that has been "humanized", i.e. by replacing one or more amino acid residues in the amino acid sequence of said naturally occurring VHH sequence by one or more of the amino acid residues that occur at the corresponding position(s) in a VH domain from a conventional 4-chain antibody from a human being. This can be performed by any suitable method known in the art such as but not limited to those disclosed by Jones et al. 1986, Riechmann et al. 1988, Presta 1992, Vaswani and Hamilton 1998, Harris 1995, Hurle and Gross 1994 and specific disclosures relating to humanization of VHHs such as e.g. Vincke et al.2009. The humanized VHH as disclosed herein can be obtained in any suitable manner known per se and thus are not strictly limited to polypeptides that comprise a naturally occurring VHH domain as a starting material.

In an embodiment of the polypeptide, as disclosed herein, the VHH has an FR1-CDR1-FR2-CDR2-FR3-CDR3-FR4 amino acid sequence structure.

In an embodiment, CDR1 exhibits between 80% and 100% sequence identity to a sequence chosen from SEQ ID N°1, SEQ ID N°2, SEQ ID N°3, or SEQ ID N°4. In another embodiment, CDR1 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°1, SEQ ID N°2, SEQ ID N°3, or SEQ ID N°4.

In an embodiment, CDR2 exhibits between 80% and 100% sequence identity to a sequence chosen from SEQ ID N°5, SEQ ID N°6, SEQ ID N°7, or SEQ ID N°8. In another embodiment, CDR2 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°5, SEQ ID N°6, SEQ ID N°7, or SEQ ID N°8.

In an embodiment, CDR3 exhibits between 80% and 100% sequence identity to a sequence chosen from SEQ ID N°9, SEQ ID N°10, SEQ ID N°11, or SEQ ID N°12. In another embodiment, CDR3 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°9, SEQ ID N°10, SEQ ID N°11, or SEQ ID N°12.

In an embodiment, FR1 exhibits between 80% and 100% sequence identity to a sequence chosen from SEQ ID N°13 or SEQ ID N°14. In another embodiment, FR1 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°13 or SEQ ID N°14.

In an embodiment, FR2 exhibits between 80% and 100% sequence identity to a sequence chosen from SEQ ID N°15, SEQ ID N°16, SEQ ID N°17 or SEQ ID N°18. In another embodiment, FR2 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°15, SEQ ID N°16, SEQ ID N°17 or SEQ ID N°18.

In an embodiment, FR3 exhibits between 80% and 100% sequence identity to a sequence chosen from SEQ ID N°19, SEQ ID N°20, SEQ ID N°21 or SEQ ID N°22. In another embodiment, FR3 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°19, SEQ ID N°20, SEQ ID N°21 or SEQ ID N°22.

In an embodiment, FR4 exhibits between 80% and 100% sequence identity to a sequence chosen from N°23 or SEQ ID N°24. In another embodiment, FR4 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from N°23 or SEQ ID N°24.

In a preferred embodiment the VHH of the peptide disclosed herein has a FR1-CDR1-FR2-CDR2-FR3-CDR3-FR4 sequence structure wherein:

- CDR1 exhibits at least 90%, more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°1, SEQ ID N°2, SEQ ID N°3, or SEQ ID N°4; and wherein
- CDR2 exhibits at least 90% more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°5, SEQ ID N°6, SEQ ID N°7, or SEQ ID N°8; and wherein
- CDR3 exhibits at least 90%, more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°9, SEQ ID N°10, SEQ ID N°11, or SEQ ID N°12; and wherein

- FR1 exhibits at least 90%, more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°13 or SEQ ID N°14; and wherein
- FR2 exhibits at least 90%, more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°15, SEQ ID N°16, SEQ ID N°17 or SEQ ID N°18; and wherein
- FR3 exhibits at least 90%, more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°19, SEQ ID N°20, SEQ ID N°21 or SEQ ID N°22; and wherein
- FR4 exhibits at least 90%, more preferably at least 95%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, more preferably at least 99.9%, more preferably 100% sequence identity to a sequence chosen from SEQ ID N°23 or SEQ ID N°24.

In an embodiment of the polypeptide as disclosed herein, the VHH has between 80% and 100% sequence identity to an amino acid sequence chosen from SEQ ID N°25, SEQ ID N°26, SEQ ID N°27, or SEQ ID N°28. In another embodiment, VHH exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, preferably 90% identity to a sequence chosen from SEQ ID N°25, SEQ ID N°26, SEQ ID N°27, or SEQ ID N°28. Preferably, VHH has a sequence according to SEQ ID N°25, SEQ ID N°26, SEQ ID N°27, or SEQ ID N°28.

In a preferred embodiment, the VHH has a sequence according to SEQ ID N°31.

The VHH specifically binds to a fibrin region that is up to 25 amino acids long. More preferably, the binding site of VHH on the fibrin is 7 amino acid long.

In some embodiments, the region of fibrin where VHH binds is comprised between the 17th and the 50th, the 18th and the 49th, the 19th and the 48th, the 20th and the 48th, the 21st and the 47th, the 22nd and the 47th, the 23rd and the 46th, the 24th and the 45th, the 25th and the 44th, the 26th and the 43rd, the 27th and the 42nd, the 28th and the 41st, the 29th and the 40th, the 30th and the 39th amino acids from the N-terminal end of the fibrin α-chain, and more preferably between the 33rd and 39th amino acids from the N-terminal end of the fibrin α-chain.

The region of fibrin where the VHH as disclosed herein specifically binds, has at least 90%, 91%, 92, 93%, 94%, 95%, 96%, 97%, 98%, 99% or 100% to an amino acid sequence chosen from SEQ ID N°32 or SEQ ID N°33. The fibrin region where VHH as disclosed herein specifically binds, has a sequence according to SEQ ID N°32 or SEQ ID N°33.

The fibrin α-chain as used through this disclosure relates to the protein having an amino acid sequence according to SEQ ID N°34.

In an embodiment of the peptide, as disclosed herein, the VHH binds to fibrin at the thrombin binding site on the fibrin polypeptide. Notably, this region encompasses the critical thrombin exosite-1 binding site, characterized by the sequence WPFCSDE (SEQ ID N°35). The binding of the VHH to this specific segment effectively blocks thrombin's interaction with its natural binding site on fibrin. This targeted inhibition is crucial as it directly interferes with thrombin's ability to bind and activate fibrin, thereby modulating the formation and stabilization of blood clots.

This selective blockade of the thrombin binding site by the VHH introduces a novel method for controlling thrombosis and managing clot-related disorders. By preventing thrombin from engaging with fibrin, the polypeptide of the disclosure restricts the growth and mechanical stability of fibrin clots, facilitating easier clot breakdown and removal. This approach leads to a reduction in the risk of significant thrombotic events such as strokes and heart attacks, offering a targeted therapeutic strategy that could surpass the efficacy and safety of existing anticoagulants which act more broadly and are often associated with an increased risk of bleeding. Moreover, the specificity of the VHH binding not only ensures an enhanced safety profile by minimizing unintended systemic anticoagulation but also allows for a more controlled and predictable therapeutic response. This is particularly advantageous in clinical settings where managing the balance between clot prevention and treatment is critical. The detailed understanding of the interaction between the VHH and the fibrin alpha chain as elucidated in this invention paves the way for the development of targeted therapies for thrombotic disease.

The thrombin binding site on fibrin was assigned to the N-terminal Aa chain at residues 27-50 and the N-terminal Bb chain at 15-25, i.e. adjacent to the thrombin cleavage sites of FPA and FPB, respectively (Hsieh K et al., 1997).

Interestingly, these fibin sites were also considered to be involved in polymerization. It was suggested that fibrin monomer competition could dislodge thrombin from the a-chain, and hence re-expose its active site in clot formation. A later crystal structure analysis of the central fibrin E domain placed thrombin's binding via exosite-I to the same Aa region, while shifting further upstream (residues 67-71) binding to the Bb chain (Pechik I et al., 2004). The VHH of the peptide disclosed herein is in agreement with this picture. The fibrinogen Bb treatment with the protease III abolishes the inhibitory effect of VHH on thrombin generation, i.e. by (partial) removal of the thrombin binding sites on fibrin. The major structural and functional changes of the fibrin formed in the presence of VHH- amorphous clumps rather than elongating fibres able to clot retraction - illustrate the importance of proper thrombin binding for a well-structured growing fibrin network.

In a further embodiment of the polypeptide, as disclosed herein, the VHH targets the thrombin exosite-1 binding site on the fibrin β-chain.

With regard to the binding of VHH to a target, the term "specific binding" or "specifically binds to" or "binds o" or is "specific for" a particular target molecule or polypeptide, e.g. an epitope on a particular polypeptide target, means binding that is measurably different from a non-specific interaction. Specific binding can be measured, for example, by determining the binding of a molecule compared to the binding of a control molecule, which generally is a molecule of similar structure that does not have binding activity. For example, specific binding can be determined by competition with a control molecule that is similar to the target, for example, an excess of non-labelled target. In one embodiment, the term "specific binding" refers to binding where the VHH binds to a particular target molecule, polypeptide or epitope on a particular polypeptide without substantially binding to any other molecule, polypeptide or epitope.

In yet a further embodiment, the peptide as disclosed herein, comprises an immunoglobulin single variable domain and an urokinase-type plasminogen activator (uPA) domain. The uPA domain acts as a potent fibrinolytic agent, facilitating the conversion of plasminogen to plasmin, which is instrumental in the breakdown of fibrin clots. This domain, derived from uPA, is smaller, more potent, and easier to produce than the corresponding domain in tissue plasminogen activator (tPA), the current standard of care for thrombolytic therapy. The uPA domain's enhanced potency may contribute to the polypeptide's superior fibrinolytic activity. Additionally, the ease of production of the uPA domain may facilitate the large-scale manufacture of the polypeptide, potentially overcoming one of the key challenges associated with tPA-based therapies.

In an embodiment, the uPA has an amino acid sequence that exhibits between 80% and 100% sequence identity to SEQ ID N°29. In another embodiment, FR3 exhibits at least 80%, 81%, 82%, 83%, 84%, 85%, 86%, 87%, 88%, 89%, 90%, 91%, 92%, 93%, 94%, 95%, 96%, 97%, 98%, 99%, or 100%, to SEQ ID N°29. Preferably, uPA has a sequence according to SEQ ID N°29.

In an embodiment of the polypeptide, as disclosed herein, the C terminus of the VHH and the N terminus of the uPA domain are linked through a linker, providing a stable and functional configuration for the polypeptide. This arrangement further provides a synergistic effect of the uPA and VHH that enhances the therapeutic performance of the polypeptide. The VHH facilitates specific binding to fibrin. This is an essential step in the treatment of thrombotic conditions as it enables the targeting of fibrin clots, the primary culprits in such conditions. On the other hand, the uPA domain is responsible for inducing fibrinolysis, a process that effectively dissolves fibrin clots. The linker serves as a bridge connecting these two functional domains. It may relate to a sequence of amino acids or other molecular structures that allow for the spatial arrangement of the two domains in a manner that optimizes their synergistic interaction.

The length of the linker may vary depending on the specific requirements of the therapeutic application. It is preferably between 5 and 50 amino acids long, more preferably between 15 and 45 amino acids, even more preferably between 10 and 40 amino acids, and most preferably between 6 and 10 amino acids. The specific sequence of the linker may also be tailored to optimize the stability, solubility, and therapeutic efficacy of the polypeptide.

In a preferred embodiment, the linker has an amino acid sequence according to SEQ ID N°30. However, any other suitable linker-amino acid sequences known in the art may be used with the polypeptide of the current disclosure, such as but not limited to (GGGGS)3 (SEQ ID N°37), G6, G8, (EAAAK)3 (SEQ ID N° 38), PAPAP (SEQ ID N° 39).

The polypeptide as disclosed herein is particularly effective in binding to fibrin while having no cross-reactivity to fibrinogen. This is due to the high specificity of the VHH domain that was designed to not bind to fibrinogen and which binds to human fibrin with an affinity 1000 times higher than to human fibrinogen. In some embodiments, VHH binds to fibrin with an affinity at least 100 times, 200 times, 300 times, 400 times, 500 times, 600 times, 700 times, 800 times, 900 times, 1000 times, 1250 times, 1500 times, 2000 times, 2500 times, or 3000 times higher than to human fibrinogen. In alternative embodiments, the VHH binds to fibrin with an affinity 10 times, 25 times, 50 times, 100 times, 200 times, 300 times, 400 times, 500 times, 600 times, 700 times, 800 times, 900 times, 1000 times, 1250 times, 1500 times, 2000 times, 2500 times, or 3000 times higher than to human fibrinogen. This high affinity for fibrin over fibrinogen leads to a more targeted delivery of the fibrinolytic enzyme to the clot, improving the efficacy and safety of the treatment.

The polypeptide as disclosed herein exhibits fibrinolytic activity in plasma or whole blood. This means that it can dissolve fibrin clots, thereby restoring normal blood flow. This increased activity can be attributed to the unique combination of the immunoglobulin single variable domain and the uPA domain, which together provide a synergistic effect in promoting fibrinolysis. The polypeptide exhibits superior fibrinolytic activity compared to conventional agents, offering an innovative contribution to anti-fibrin therapy. Its enhanced potency for plasmin generation and overall stability contribute to its improved performance, making it a promising candidate for acute thrombosis treatment.

The fibrinolytic activity of the polypeptide as disclosed herein is 1000 times higher than the fibrinolytic activity of uPAs. Surprisingly, the addition of VHH to the uPA provides a synergistic effect which boosts the activity of the enzyme promoting thrombolysis. This elevated fibrinolytic activity results in more effective therapy, enabling quicker and more efficient resolution of thrombosis or embolism. In some embodiments, the polypeptide has a fibrinolytic activity that is at least 100 times, 200 times, 300 times, 400 times, 500 times, 600 times, 700 times, 800 times, 900 times, 1000 times, 1250 times, 1500 times, 2000 times, 2500 times, or 3000 times higher than the fibrinolytic activity of uPAs. In alternative embodiments, the polypeptide has a fibrinolytic activity that is 10 times, 25 times, 50 times, 100 times, 200 times, 300 times, 400 times, 500 times, 600 times, 700 times, 800 times, 900 times, 1000 times, 1250 times, 1500 times, 2000 times, 2500 times, or 3000 times higher than the fibrinolytic activity of uPAs.

The fibrinolytic activity was determined by measuring the fibrin generation potential of the polypeptide as disclosed herein. Measurements revealed that to generate an equivalent amount of plasmin, 10 $\mu$M of uPA was required when no nanobody was employed. However, only 10 nM of the VHH-uPA polypeptide, as described in this disclosure, was necessary to achieve the same level of plasmin generation.

In a further embodiment, the polypeptide as disclosed herein has a fibrinolytic activity higher than the tPA. This activity is at least 10 times, 9 times, 8 times, 7 times, 6 times, 5 times, 4 times, 3 times, 2 times, or 1 time higher than the fibrinolytic activity of tPA. Alternatively, the fibrinolytic activity is affinity at least 100 times, 200 times, 300 times, 400 times, 500 times, 600 times, 700 times, 800 times, 900 times, 1000 times, 1250 times, 1500 times, 2000 times, 2500 times, or 3000 times higher than the fibrinolytic activity of tPA. In yet another alternative embodiment, the fibrinolytic activity is 10 times, 25 times, 50 times, 100 times, 200 times, 300 times, 400 times, 500 times, 600 times, 700 times, 800 times, 900 times, 1000 times, 1250 times, 1500 times, 2000 times, 2500 times, or 3000 times higher than the fibrinolytic activity of tPA.

In an embodiment of the polypeptide as disclosed herein, said polypeptide exhibits thrombolytic activity. This fusion of a fibrin-specific binder (VHH) with a potent fibrinolytic enzyme (uPA) delivers a higher concentration of the enzyme to the site of a clot, leading to enhanced thrombolytic effectiveness. This thrombolytic activity of the polypeptide is indicative of the potential benefits it may offer in efficiently and specifically resolving blood clots within the body. Notably, this thrombolytic activity is not confined to a specific range but can be observed across a broad spectrum of physiological conditions. This broad-spectrum activity may enhance the versatility of the polypeptide as a therapeutic agent, as it may be effective in treating a variety of thrombotic conditions.

The thrombolytic activity of the polypeptide is preferably higher than that of conventional thrombolytic agents. For instance, the thrombolytic activity may be at least 10% higher, more preferably at least 20% higher, even more preferably at least 30% higher, and most preferably at least 40% higher than that of conventional thrombolytic agents. This enhanced thrombolytic activity may result in improved clinical outcomes, including faster resolution of blood clots and reduced risk of complications related to thrombosis.

In an alternative embodiment, the current invention relates to a polypeptide characterized exclusively by the inclusion of a VHH domain. This domain retains the identical structure, sequence, and biological function as the VHH domains detailed in the previous embodiments of the invention, where the polypeptide includes additional functional domains. Furthermore, this singular-domain polypeptide is applicable for the same therapeutic and diagnostic purposes, and it is manufactured using the same processes as its multi-domain counterparts, as set forth in the various embodiments described herein.

**Pharmaceutical compositions**

The polypeptide as disclosed herein and described in the previous embodiments, may be included in a composition comprising a pharmaceutical-acceptable carrier, excipient or diluent.

A carrier is considered as being pharmaceutically acceptable, when it does not have any or not substantially adverse unwanted effects on the human or animal body, e.g. it is considered generally safe, nontoxic and/or does not cause unwanted biological side reactions. Suitable pharmaceutically acceptable carriers are well known to the person skilled in the art. The choice of carrier may depend upon the route of administration and concentration of the polypeptide and the carrier may be in the form of a lyophilised composition or an aqueous solution. Generally, an appropriate amount of pharmaceutically acceptable salt is used in the carrier to render the composition isotonic. Examples of the carriers include but are not limited to saline, Ringer's solution and dextrose solution. Preferably, acceptable excipients, carriers, or stabilisers are non-toxic at the dosages and concentrations employed, including buffers such as citrate, phosphate, and other organic acids; salt-forming counter-ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). The composition may also optionally include other components, such as buffering agents or stabilizing agents.

Preferably, the composition for parenteral administration is prepared in a sterile environment and is formulated as a solution, a suspension, an emulsion, a lyophilized powder for reconstitution, or other suitable forms for parenteral administration.

The composition comprising the polypeptide as disclosed herein is preferably formulated for parenteral administration. The administration of the peptide or composition via parenteral routes includes but is not limited to, intravenous, intramuscular, subcutaneous, or intraperitoneal routes. The parenteral administration of the composition allows for the rapid delivery of the therapeutic agent directly into the systemic circulation, bypassing the gastrointestinal tract and potential enzymatic degradation or alteration. This rapid delivery is especially advantageous in acute thrombotic events, where time is of the essence and rapid therapeutic action can improve patient outcomes significantly.

In an embodiment, the composition may be administered in a single dose or multiple doses depending on the severity of the disease or condition characterized by a thrombotic or embolic state. The dosage may be adjusted according to the weight, age, sex, and overall health condition of the subject. The exact dosage and frequency of administration may be determined by a healthcare provider based on clinical judgment and patient response to treatment.

The content of the polypeptide in the pharmaceutical composition is not limited as far as it is useful for treatment, prevention, or amelioration but preferably contains 0.0000001 -10% by weight per total composition

## Methods of treatment

The current disclosure relates to methods of treatment and prevention of various diseases using the polypeptide or a composition comprising the polypeptide of the invention, wherein the polypeptide comprises an uPA domain and a VHH domain which can bind to fibrin via a fibrin-binding domain.

In an embodiment, the current disclosed related to the peptide or composition comprising said peptide according to any of the previous embodiments, for use in the treatment, prevention or amelioration of a disease or condition characterized by a thrombotic or embolic state in a subject in need thereof.

The peptide or composition as disclosed herein, are used for the treatment, prevention or amelioration of a disease or condition disease or condition characterized by a thrombotic or embolic state selected from embolism, lung embolism, stroke, infarction, brain infarction, thrombotic stroke, a microthrombotic disorder, venous occlusive disease, ischemia, thrombosis in dialysis patients, acute myocardial infarction, deep vein thrombosis, acute ischemic stroke, acute peripheral arterial occlusion, occlusion of indwelling catheters, intracardiac thrombus formation and microthrombotic ischemia.

In a further embodiment, the peptide or composition as disclosed herein is for use in emergency medicine, or wherein the disease or condition is an acute or chronic disease or condition.

In a most preferred embodiment, the composition may be administered in emergency situations, where immediate fibrinolytic and thrombolytic activity is required to prevent or treat life-threatening thrombotic or embolic events, such as stroke, myocardial infarction, or pulmonary embolism. The rapid therapeutic action of the composition upon parenteral administration provides a significant advantage in these critical situations, potentially saving lives and improving patient outcomes.

For example, the polypeptide or pharmaceutical composition comprising the polypeptide as disclosed herein can be administered to a patient diagnosed with acute thrombotic or embolic state and risk factors for residual emboli or diagnosed to be at risk of having an acute thrombotic or embolic state. For example, the polypeptide or pharmaceutical composition comprising the polypeptide as disclosed herein can be administered within 10 minutes, 20 minutes, 30 minutes, 60 minutes, 2 hours, 3, 4, 5, 6, 7, 8, 9, 10, 12 or 24 hours after being diagnosed with acute thrombotic or embolic state and risk factors for residual emboli or being diagnosed to be at risk of having an acute thrombotic or embolic state.

The polypeptide or pharmaceutical composition comprising the polypeptide as disclosed herein can be administered by any suitable route of administration including oral administration and parenteral administration such as intranasal, subcutaneous, intravenous, intraarterial, intracardial and intramuscular administration. Preferably, the polypeptide or pharmaceutical composition comprising the polypeptide as disclosed herein is administered parenterally. Therefore, in one embodiment, the polypeptide or pharmaceutical composition can be formulated for parenteral administration. In a preferred embodiment, the polypeptide or pharmaceutical composition is formulated for parenteral administration, in particular, selected from intravenous, intraarterial, intracardiac, intradermal, subcutaneous, intraembolic, intramucosal or intraarticular administration. For example, the polypeptide or pharmaceutical composition can be formulated for intravenous administration, such as intravenous infusion or injection. In another preferred embodiment, the polypeptide or pharmaceutical composition is administered by parenteral administration, in particular, selected from intravenous, intraarterial, intracardiac, intradermal, subcutaneous, intraembolic, intramucosal or intraarticular administration. For example, the polypeptide or pharmaceutical composition is administered by intravenous administration, such as intravenous infusion or injection.

In a further embodiment the VHH, compound, pharmaceutical composition, or medical device for use as described herein is used in combination with a further thrombolytic agent. The further thrombolytic agent is preferably selected from a tPA protein, streptokinase, alteplase, reteplase, tenecteplase, urokinase, prourokinase, and anistreplase (APSAC). These thrombolytic agents are well-known in the art and are approved or used as thrombolytic agents. The further thrombolytic agent can be administered to a subject at a lower dose in the combination comprising the polypeptide of the invention and a second thrombolytic agent as compared to the administration of the second thrombolytic agent alone. For example, the dose may be lowered by 10%, 20%, 30%, 40%, 50% or 90%. For example, the further thrombolytic agent can be administered spatially separately with the polypeptide or pharmaceutical composition or together, such as a single composition, vial, container, kit or kit-of-parts. For example, the further thrombolytic agent can be administered temporally separately from the polypeptide or pharmaceutical composition, or at the same time point, and/or within 1, 2, 3, 4, 5, 6, 7, 8, 9, 10 or 30 minutes or within 1, 2, 3, 4, 5, 6, 7, 8, 9, 10, 12, 24 or 48 hours.

The disclosure further relates to a method of treating, preventing or ameliorating a disease or condition characterized by a thrombotic or embolic state in a subject in need thereof, comprising administering to said subject a pharmaceutically effective amount of the polypeptide or of the pharmaceutical composition.

As used herein, the term "pharmaceutically effective amount" in the context of the administration of a therapy to a subject refers to the amount of a therapy that achieves a desired therapeutic, preventive/prophylactic or ameliorating effect.

A suitable amount and dosage can be determined by persons skilled in the art. For example, a polypeptide or pharmaceutical composition described herein may administered to a subject (e.g., via intravenous injection) at about 0.001 mg/kg, 0.01 mg/kg 0.1 mg/kg, 0.3 mg/kg, 1 mg/kg, 3 mg/kg, 6 mg/kg, or about 10 mg/kg. Thereby, administering of the polypeptide or pharmaceutical composition of the present invention refers to any route of drug administration known to the person skilled in the art, such as parenteral, intravenous, intraperitoneal, subcutaneous, oral, intranasal or sublingual administration. Suitable dosage regimens are also well known to the person skilled in the art. Preferably, the polypeptide or pharmaceutical composition of the present invention is administered in a pharmaceutically effective amount, i.e. in a dose or concentration causing a biological response in the body the polypeptide or pharmaceutical composition is administered to.

## Diagnostic methods

The current disclosure also relates to diagnostic methods using the peptide disclosed in the previous embodiments. In a preferred embodiment, the polypeptide comprising the VHH and the uPA, or a composition comprising said peptide is used in the diagnosis of abnormal fibrinolytic states in bleeding and thrombotic disorders.

The polypeptide as disclosed herein demonstrates a significant advancement in the calibrated, automated plasmin generation (PG) assay, enhancing the activation of plasminogen in the presence of fibrin and increasing the assay's sensitivity to plasminogen activator inhibitor-1 (PAI-1). The polypeptide binds fibrin with high specificity and facilitates the precise measurement of fibrinolytic parameters such as lag time, time to peak, endogenous plasmin potential, and peak plasmin generation. The diagnostic capability of the aFn-uPA augmented PG assay to detect variations in these parameters, especially in response to PAI-1, allows for a detailed evaluation of a patient's fibrinolytic system, distinguishing between hyperfibrinolytic and hypofibrinolytic conditions. Furthermore, the modulation of the assay by anti-PAI-1 antibodies elucidates the functional impact of PAI-1 and its potential as a therapeutic target. Hence, the invention provides a robust and sensitive diagnostic approach, utilizing this polypeptide, for the assessment and monitoring of fibrinolytic activity, which is vital in the clinical management of patients with conditions characterized by impaired fibrinolysis, delivering a valuable tool in both diagnostic and therapeutic landscapes.

## Nucleic acids, vectors, host cells

The current disclosure also relates to nucleic acids encoding the polypeptides of the invention, to vectors comprising said nucleic acids and to host cells comprising the nucleic acids encoding the polypeptide of the invention or comprising the vectors comprising the nucleic acids encoding the polypeptide.

The term "nucleic acid" describes any form of deoxyribonucleic acid (DNA), ribonucleic acid (RNA) or artificial nucleic acid known to the person skilled in the art. Nucleotide sequences encoding the VHH, uPA or polypeptide disclosed herein, and modified versions of these are determined using methods well known in the art, i.e. nucleotide codons known to encode particular amino acids are assembled in such a way to generate a nucleic acid that encodes the VHH, uPA or polypeptide. Such a polynucleotide encoding the VHH, uPA or polypeptide can be assembled from chemically synthesized oligonucleotides, which, briefly, involves the synthesis of overlapping oligonucleotides containing portions of the sequence encoding the VHH, uPA or polypeptide, annealing and ligating of those oligonucleotides, and then amplification of the ligated oligonucleotides by PCR. A nucleic acid encoding the VHH, uPA or polypeptide can be chemically synthesized or obtained from a suitable source (e.g., cells selected to express the VHH, uPA or polypeptide described herein) by PCR amplification using synthetic primers hybridizable to the 3' and 5' ends of the sequence or by cloning using an oligonucleotide probe specific for the particular gene sequence to identify, e.g., a DNA clone that encodes the VHH, uPA or polypeptide. Amplified nucleic acids generated by PCR can then be cloned into replicable cloning vectors using any method well-known in the art.

DNA coding for the VHH, uPA or polypeptide of the invention described herein can be readily isolated and sequenced using conventional procedures (e.g., by using oligonucleotide probes that are capable of binding to sequences encoding FR1 and FR4 regions. Once isolated, the DNA can be placed into expression vectors, which are then transfected into host cells such as E. coli cells, simian COS cells, Chinese hamster ovary (CHO) cells (e.g., CHO cells from the CHO GS System™ (Lonza)), or myeloma cells that do not otherwise produce the VHH, uPA or polypeptide, to obtain the synthesis of the VHH, uPA or polypeptide in the recombinant host cells.

The VHH, uPA or polypeptide are amplified using PCR primers. For example, PCR primers including VHH variable domain nucleotide sequences, a restriction site, and a flanking sequence to protect the restriction site can be used to amplify the VHH sequences. Utilizing cloning techniques known to those of skill in the art, the PCR amplified VHH domain, uPA domain or the entire polypeptide, can be cloned into vectors, which optionally express further domain(s) such as a purification tag, as in the examples, or a protein providing for an extended half-life in vivo. In certain embodiments, the expression vectors comprise a promoter, a secretion signal, a cloning site for the variable region, and a selection marker such as neomycin. The vectors are then transfected into cell lines to generate stable or transient cell lines that express the VHH, the uPA or the polypeptide, using techniques known to those of skill in the art.

Site-directed or high-density mutagenesis of the variable region or other mutagenesis methods can be used to optimize the specificity, affinity, etc. of the VHH. In particular, affinity maturation strategies are known in the art and can be employed to generate high-affinity VHH.

Preferably, the nucleic acid is part of a vector. Vectors are plasmids which are used to introduce a desired nucleic acid sequence, such as a gene, into a target cell, resulting in the transcription and translation of the protein encoded by the nucleic acid sequence, e.g. the VHH, the uPA or the polypeptide disclosed herein. Therefore, the expression vector in general comprises regulatory sequences, such as promoter and enhancer regions, as well as a polyadenylation site in order to direct efficient transcription of the nucleic acid sequence on the expression vector. The expression vector may further comprise additional necessary or useful regions, such as a selectable marker for selection in eukaryotic or prokaryotic cells, a purification tag for the purification of the resulting protein, a multiple cloning site or an origin of replication. The expression vector may be a viral or a non-viral vector. In general, various kinds of viral vectors, such as retroviral vectors, e.g. lentiviral or adenoviral vectors, or plasmids may be used. A vector comprising the nucleic acid encoding thr VHH, uPA or polypeptide disclosed herein may further be introduced in a host cell.

Methods for introducing such a vector into a host cell are well known to the person skilled in the art, such as any known transfection method, e.g. any nonviral transfection method (e.g. chemical-based, non-chemical or particle-based) or any virus-based transfection method. Examples of suitable methods are transfection methods based on calcium phosphate precipitation, lipofection, cationic polymers, Fugene, Dendrimer, nanoparticles, microinjection, cell squeezing, electroporation, particle gun (also known as gene gun), magnet-assisted transfection, optical transfection, protoplast fusion, hydrodynamic delivery, sonoporation, transferrin-based infection, antibody-mediated transfection or virus-based transfection (e.g. based on adenoviral or lentiviral vectors).

Suitable host cells are well known to the person skilled in the art, such as mammalian cells (such as human, mouse, rat or hamster cells), insect cells, bacterial cells or yeast cells. Such host cell may comprise a nucleic acid of the present invention e.g. integrated in its genome or in a vector. Methods for introducing such nucleic acid in the host cell are described above and further well-known to the person skilled in the art.

However, the invention is not limited to this application. The peptides, compositions, and methods detailed herein are applicable across various fields where fibrin binding is relevant.

The invention is further described by the following non-limiting examples which further illustrate the invention, and are not intended to, nor should they be interpreted to, limit the scope of the invention.

## EXAMPLES

### Example 1

**Materials and Methods**

**Materials**

Recombinant tissue factor (Innovin) was purchased from Siemens Healthineers (Marburg, Germany). Fluorogenic substrate Z-Gly-Gly-Arg-aminomethyl coumarin (Z-GGR-AMC) came from Bachem (Basel, Switzerland). Thrombin calibrator, $\alpha_2$-macroglobulin-thrombin complex, human thrombin, protease III, thrombomodulin and active protein C were prepared by Synapse Research Institute (Maastricht, The Netherlands). Alexa Fluor 546-labeled human fibrinogen was purchased from Invitrogen Life Technologies (Budapest, Hungary). Ancrod was obtained from Knoll (Ludwigshafen, Germany). Chromogenic substrate S2238 was from Chromogenix (Brussels, Belgium). The chromogenic substrate staphylocoagulase was from Fresenius Kabi (Uppsala, Sweden). The peptide GPRP was purchased from Bachem Inc. (Torrance, CA, USA). Normal pool plasma (NPP) was prepared in house. Antithrombin-deficient plasma was obtained from Hematologic Technologies LLC (Essex Junction, VT, USA). Fibrinogen-deficient plasma and factor IX, XI or XII deficient plasmas were from Affinity Biologicals (Ancaster, Canada). Nanobody Nb106 was generated in-house from a llama injected with fibrin degradation products, according to procedures as indicated before. In characterization studies, it was selected as fibrin monomer binding, but non-fibrinogen binding. The non-responsive nanobody Nb-Syn1C6 served as a negative control. Glycoprotein VI (GPVI) agonist, collagen-related peptide cross-linked (CRP-XL) was from CambCol (Cambridge, United Kingdom); ionomycin was from Calbiochem (San Diego, CA, USA); thrombin receptor-activating peptide 6 (SFLLRN, TRAP6) from Bachem (Bubendorf, Switzerland). The direct oral anticoagulant dabigatran was purchased from Alsachim (Illkirch Graffenstaden, France).

**Blood collection and sample preparation**

The study was approved by the Medical Ethical Committee of Maastricht University Medical Center and was conducted according to the Declaration of Helsinki. The patient study was approved by the Research and Ethics Committee of Padua University Hospital. Blood was taken from the antecubital vein of subjects, who were not taking anticoagulant or antiplatelet medication. Blood samples (9 volumes) were drawn into aseptic vacutainer tubes (Greiner Bio-One, Kremsmunster, Austria), containing 3.2% sodium citrate (1 volume). Samples were kept at room temperature, and used within 2-3 h. Blood cell parameters were measured with a Coulter counter analyzer (Beckman Coulter, Woerden, The Netherlands). The platelet count of all healthy subjects was in the normal range, $150\text{-}400 \times 10^9/L$.

Platelet-poor plasma (PPP), platelet-rich plasma (PRP) and washed platelets were prepared, basically as described before. In brief, PRP was obtained by centrifuging blood for 15 min at 250 g; PPP was obtained by centrifuging twice for 10 min at 2630 g. For collecting washed platelets, PRP was supplemented with 10 vol% ACD medium (80 mM trisodium citrate, 52 mM citric acid and 180 mM glucose), after which the cells were spun down in 2 mL Eppendorf tubes at 1700 g for 2 min. After the removal of plasma, the pellets were resuspended into 1 mL of Hepes buffer pH 6.6 (10 mM Hepes, 136 mM NaCl, 2.7 mM KCl, 2 mM $MgCl_2$, apyrase at 0.2 unit ADPase/mL, 0.1% glucose and 0.1% bovine serum albumin). After another addition of 6.6 vol% ACD, the tubes were recentrifuged, and the washed platelets were resuspended into 1 mL Hepes buffer pH 7.45 (10 mM Hepes, 136 mM NaCl, 2.7 mM KCl, 2 mM $MgCl_2$, 0.1% glucose, and 0.1% bovine serum albumin). PPP were also used from 64 healthy subjects, as described before. Samples were obtained from 28 males and 36 females, with mean ages of 34 and 32, respectively.

For mouse experiments, blood was obtained by orbital puncture from adult C57BL/6 mice (males and females), as described. Blood samples were collected into 3.2% citrate, and used for microfluidic testing and plasma preparation. Permission was obtained from the Animal Experimental Ethics Committee, with approval from the District of Lower Franconia (Germany).

**Thrombin generation measurements**

Calibrated automated thrombin generation in PPP or PRP was performed in 96-well plates, following an earlier described procedure. In brief, wells contained 80 $\mu$L PPP or PRP and 20 $\mu$L of trigger solution: 1 pM tissue factor and 4 $\mu$M procoagulant phospholipids, dissolved into BSA5 buffer (20 mM Hepes, 140 mM NaCl, 0.5% bovine serum albumin, pH 7.35). The reaction was started by dispensing 20 $\mu$L of substrate solution [11 mM $CaCl_2$, 5.5 mM $MgCl_2$ and 417 $\mu$M Z-GGR-AMC, dissolved into BSA60 buffer (20 mM Hepes, 6% bovine serum albumin, pH 7.35)]. For each plasma sample, a calibrating well was included, where the trigger solution was replaced by calibrator (corresponding with 320 nM thrombin activity). The calibration curves corrected for effects of plasma color, and allowed to convert fluorescence levels into nM thrombin concentrations. Curve parameters automatically obtained were: thrombin lagtime (min), thrombin peak height (peak, nM), time to peak (Ttpeak, min), curve velocity index (VI, nM/min), and endogenous thrombin potential (ETP, nM.min). Samples in wells were pre-incubated for 10 min at 37 °C with indicated modulators of thrombin or fibrin generation.

For whole blood thrombin generation, citrated blood samples were mixed with substrate solution (Z-GGR-AMC dissolved into BSA60 buffer) and trigger solution (1 pM tissue factor, 6 mM $CaCl_2$ and 3 mM $MgCl_2$ dissolved into BSA5 buffer). The volume ratio of blood, trigger solution and substrate solution was 3:2:1. A modified Excel-based calculation template was used to automatically generate first derivative thrombin generation curves from the raw fluorescence data. Full curve parameters obtained were, as described above.

**Coagulation measurements**

Plasma clotting times were measured using a STart apparatus (Stago, Paris, France). Herein, plasmas were triggered with 1 pM tissue factor, 4 $\mu$M procoagulant phospholipids, 11 mM $CaCl_2$ and 5.5 mM $MgCl_2$. Fibrin clotting was also monitored over time in 96-well plates by spectrometric recording of changes in optical density (turbidity) at 405 nm. Again, samples of PPP or PRP were mixed with 1 pM tissue factor, 4 $\mu$M phospholipids, 11 mM $CaCl_2$ and 5.5 mM $MgCl_2$.
Fibrinogen activity levels (Clauss method, expressed as g/L, reference range 1.5-4.5 g/L) and fibrinogen antigen levels (reference range 80-120%) were determined as before.

**Clot retraction**

To measure clot formation and retraction, samples of whole blood or PRP were diluted, as appropriate into Hepes buffer pH 7.45. Coagulation was triggered by the addition of 1 pM tissue factor and 10 mM $CaCl_2$, as described. Experiments were conducted in siliconized glass tubes at 37 °C for up to 150 min. Clots were imaged at indicated times.

**Light transmission and scanning electron microscopy**

Normal pool plasma (NPP) or anti-thrombin III deficient plasma (ATDP) was preincubated with Nb106 (100 $\mu$g/mL) or vehicle (control) for 10 minutes at 37°C. Then, fibrin formation was triggered with 1 pM tissue factor, 4 $\mu$M phospholipids, 11 mM $CaCl_2$, and 5.5 mM $MgCl_2$. After thorough mixing, the mixture was immediately transferred to a glass slide and the fibrin formation was observed with an inverted light transmission microscope with 63x objective (Leica DFC 3000 G, Wetzlar, Germany). Images of the central area and edge area were taken at 5, 10, 15, 20 and 30 min. The condition is the same as in the thrombin generation assay. The pictures of the edge area are not shown. Mouse fibrin structures were imaged as before.
For high-resolution imaging, fibrin meshes were formed on a Sefar sieve (mesh pores 170 $\mu$m) in 96-well plates. The clots formed after 30 min were fixed with 4% paraformaldehyde for 1 h. After washing with phosphate-buffered saline, samples were dehydrated with a gradient of ethanol (30% to 100%), followed by hexamethyl disilazane/ethanol (1:1) and 100% hexamethyl disilazane. After drying for 1 h, the samples were mounted onto aluminium pin studs with 12 mm carbon conductive tabs (Ted Pella, Redding, CA, USA), and were sputter coated with gold (Quorum Technologies, Ashford, United Kingdom), and then imaged with a Phenom G2 Pro desktop scanning electron microscope (Phenom World, Eindhoven, The Netherlands) using Phenom ProSuite software.

**Prothrombin consumption measurements**

Plasma samples were analyzed before and after completion of thrombin generation under conditions where ETP was measured and quantified as nM x min levels of thrombin activity. The 96-well plate-based measurement relies on the addition of a molar surplus of staphylocoagulase (from *Staphylococcus aureus*), which binds to human prothrombin and exposes its catalytic site, the activity of which is measured with chromogenic substrate S2238. As described elsewhere, 25 mL of 1:400 prediluted plasma samples, were supplemented with 25 $\mu$L staphylocoagulase (50 nM), and incubated at 37 °C for 10 min. The mixture was supplemented with S2238 in Tris buffer pH 7.9, and substrate cleavage was measured at 405 nm for 5 min in a plate reader. Measurements were compared with calibrator wells and a negative control without staphylocoagulase. The lifetimes of each pool were calculated by the formulas below:

$$\text{Fr. Pool 1} = \frac{\text{ETP}_{\text{Nb106}}}{\text{ETP}_{\text{Con}}} \qquad (1)$$

$$\text{Fr. Pool 2} = 1 - \frac{\text{ETP}_{\text{Nb106}}}{\text{ETP}_{\text{Con}}} \qquad (2)$$

$$\text{PC (Prothrombin consumption)} = \text{Prothrombin}_{\text{plasma}} - \text{Prothrombin}_{\text{serum}} \qquad (3)$$

$$\text{LT( life time) in pool 1} = \frac{\text{ETP}_{\text{Nb106}}}{\text{PC}_{\text{Nb106}}} \qquad (4)$$

$$\text{LT (life time) in pool 2} = \frac{\frac{ETP_{Con}}{PC_{Con}} - \text{LT in pool 1} \times \text{Fr. Pool 1}}{\text{Fr. Pool 2}}$$

(5)

### Microfluidics of platelet-fibrin thrombus formation

Platelet-dependent fibrin formation in whole blood under flow conditions (wall shear rate 200 or 1000 s$^{-1}$) on microspots of Horm collagen type I was assessed, as described before for human blood and mouse blood. Prior to perfusion, citrate-anticoagulated blood samples were pre-incubated with a control vehicle or Nb106, Alexa Fluor 649-labeled anti-GPIX antibody 56F8, and Alexa Fluor 488-labeled fibrinogen. Coagulation was achieved by co-infusion of the blood 10:1 with trigger solution (1 pM tissue factor, 3.75 mM $MgCl_2$ and 7.5 mM $CaCl_2$, f.c.). Brightfield and fluorescence images were acquired over time. Blinded image quantification was for surface area coverage using semi-automated Image J scripts.

### Statistics

Statistical analyses were performed with GraphPad Prism 8 (San Diego, CA, USA). Data were generally presented as mean $\pm$ SD. Significances were determined using student t-test, one-way ANOVA or two-way ANOVA, as required. Values of $p < 0.05$ were considered as statistically significant.

### Results

### *Separating fibrin-dependent and fibrin-independent thrombin generation*

For re-investigating the complex interactive roles of fibrin and thrombin, we used a new nanobody, Nb106, generated in-house from a llama injected with fibrin degradation products. The nanobody Nb106 was characterized as binding to human fibrin (monomers), but not to un-cleaved fibrinogen. Surprisingly, we observed a major dose-dependent effect of Nb106 on tissue factor-induced thrombin generation, which was similar in extent when performed in PPP, PRP or whole blood from a single donor (Figure 1A). In thrombin generation, the activation and later inactivation of thrombin are continuously monitored from the fluorescence signal caused by cleavage of its peptide substrate Z-GGR-AMC. In the calibrated, first-derivative thrombin generation curves, we found increasing inhibitory effects up to a concentration of 100 $\mu$g/mL (7.5 mM) Nb106, which is equimolar to fibrinogen (2.1-3.1 g/L, 6.1-9.2 mM) in normal plasmas. In comparison, an indifferent control nanobody Nb-Syn1C6 at this dose was without effect in plasma and whole blood. Quantification of curves with whole blood from multiple donors showed a minimal effect of Nb106 (100 $\mu$g/mL) on the thrombin lagtime (91.21$\pm$1.55% of control), but major reductions in thrombin peak height (71.26$\pm$0.32%) and endogenous thrombin potential (ETP or area-under-the-curve, representing the integrated activity of thrombin formed over time; 46.92$\pm$0.74%). For PPP, Nb106 similarly did not affect the lagtime (89.46$\pm$6.43%) but suppressed peak height (43.70$\pm$2.92%) and ETP (38.34$\pm$0.16%). The overall dose-response analysis of curve parameters confirmed that Nb106 was similarly suppressive on the extent of thrombin generation in whole blood, PRP and PPP (Figure 1B-D).

The observed lack of effect on thrombin lagtime was the first indication that Nb106 did not affect the initiation phase, but interfered later in the coagulation process. To further investigate this, the nanobody was spiked into plasma samples at later time points, *i.e.* at 5-30 min after triggering with tissue factor. Examination of the raw fluorescence curves (Figure 2A) and of the quantified fluorescence at 50 min (Figure 2B) showed a gradual diminishment of the nanobody effect upon later addition, with no effect left at 30 min. The results suggested a main interference during first 10-min, *i.e.* the phase of fibrin formation.

To find the inhibitory mechanism, we used several factor-deficient human plasmas to test how the Nb106 interfered with thrombin generation (Figure 3). In parallel, we supplemented the plasmas with platelets, to check the nanobody's effect in the reconstituted PRP (Figure 14). We found that Nb106 had a major reducing effect with normal pool plasma (NPP), and with plasmas deficient in factor IX, factor XI or factor XII (Figure 3A-D). In contrast, it no longer reduced the thrombin generation of plasmas deficient in fibrinogen or antithrombin (Figure 3E-F). This was statistically confirmed by quantitative analysis of the curve parameters, thrombin lagtime, peak height and ETP (Figure 3G). Similarly, in the presence of platelets, Nb106 no longer suppressed the thrombin generation process when fibrinogen or antithrombin were absent (Figure 14A-G). Hence, its interference relied on the presence of both fibrin(ogen) and antithrombin.

Inspection of the raw data, obtained with NPP (Figure 15A) and deficient plasmas (2B, C), showed parallel fluorescence traces for the vehicle control and Nb106 conditions (ii). In first-derivative curve parameters (iii) and equation-fitted curves (iv)I, this appeared as a lack of Nb106 effect on the thrombin lagtime. In addition, we found similar traces of the raw data when supplemented with platelets, and no substrate consumption (data was not shown). Together, this indicated that the nanobody did not interfere with the primary mechanisms of thrombin generation and inactivation.

To establish the binding site of Nb106 on fibrin, we used several substances known to interfere with the formation of fibrin: (a) protease III from the snake *Crotalus atrox,* which cleaves the fibrinogen-Bβ chain N-terminally also releasing FBP; (b) the protease ancrod cleaving the Aa chain C-terminally;[42] (c) the fibrin polymerization-inhibiting peptide GPRP; and (d) the factor XIII inhibitor dansyl cadaverine.

Prior plasma treatment with protease III reduced tissue factor-induced thrombin generation in such a way that the inhibitory effect of Nb106 became annulled (Figure 4A). On the other hand, plasma pre-treatment with ancrod affected thrombin generation neither in the absence or the presence of Nb106 (Figure 4B), while the pre-treatment with GPRP was inhibitory without further reducing the effect of Nb106 (Figure 4C). Plasma factor XIII inhibition with dansyl cadaverine (25-250 mM) also did not alter the nanobody effect (not shown). These results provided evidence that the nanobody's effect relied on the presence of the N-terminal region of the Bb chain, exposed after the release of FPB. Since this region also binds exosite-1 of thrombin, leaving open its catalytic site, we concluded that the nanobody competed with thrombin for binding to fibrin, thus inducing the release of thrombin into the soluble plasma. Accordingly, the effect on thrombin generation may be caused by making the fibrin-bound thrombin more accessible to antithrombin inactivation by bringing it back into the plasma. By implication, Nb106 may shorten the average thrombin lifetime in activated plasma.

In calibrated tests of thrombin generation, the ETP represents the integrated activity of thrombin regarding substrate cleavage overtime. Accordingly, by measuring both the ETP and prothrombin consumption in an activated plasma sample, the average lifetime of active thrombin molecules can be calculated. We performed these measurements in parallel with normal pool plasma and antithrombin-deficient plasma in the presence and absence of Nb106, using tissue factor as a common trigger. Interestingly, while the Nb106 in normal plasma greatly reduced the ETP, it did not affect prothrombin consumption of about 1 mM (Figure 5A-B). On the other hand, in antithrombin-deficient plasma, the 3 times higher ETP level was not affected by Nb106, but the consumed prothrombin amount was about 0.5 mM. We then defined the ETP reduction by Nb106 as an estimate of the fraction of fibrin-bound thrombin in plasma. The Nb106-insensitive fraction (fibrin-independent in NPP) was assigned as thrombin pool 1, and the remaining Nb106-sensitive fraction (fibrin-dependent in NPP) as thrombin pool 2 (Figure 5C). For normal plasma, calculations resulted in thrombin lifetimes for pools 1 and 2 as $1.21 \pm 0.13$ and $2.70 \pm 0.45$ min, respectively (means $\pm$ SD, n=5). For antithrombin-deficient plasma, the overall calculated thrombin lifetime was increased to $11.98 \pm 0.17$ min (Figure 5D). These values are comparable to earlier measured half-life times of thrombin spiked into normal and antithrombin-deficient plasmas.

Collectively, these results pointed to a 2.2-fold increase in the lifetime of the thrombin pool on fibrin that is eliminated by Nb106, and to a 9.9-fold increase in thrombin lifetime in the absence of antithrombin. This supports our deduction that both a soluble and fibrin-bound thrombin pool contributes to the thrombin generation process and that the Nb106-replaceable thrombin on fibrin is temporarily protected against antithrombin inactivation.

### *Variables of fibrin-dependent thrombin generation*

Given the unclarity of the fibrinogen level as a determinant of thrombin generation, we examined the effects of Nb106 on this process using tissue factor as a common trigger. We first tested a dose range of Nb106 (0-150 μg/mL) in fibrinogen-deficient plasma reconstituted with 0-4.10 g/L fibrinogen. In all plasmas with fibrinogen, the addition of Nb106 inhibited the thrombin generation traces in a dose-dependent way (Figure 6A-E). A constructed heatmap for the ETP values showed a tendency to lower the absolute inhibition above 2.9 g/L fibrinogen (Figure 6F), which is in accordance with a sub-equimolar amount of nanobody added in the presence of the highest fibrinogen concentrations.

We then tested the higher concentration (150 μg/mL) of Nb106 with plasmas from a cohort of 64 healthy subjects. As indicated in Figure 7A, the Nb106 did not affect the thrombin lagtime or time-to-peak value, but it consistently suppressed both peak height and ETP level. When expressed as a function of the plasma fibrinogen level (Clauss method), the relative inhibition varied by $52.35 \pm 0.14\%$ for peak height and $54.91 \pm 0.12\%$ for ETP (mean $\pm$ SD, n = 64). Again, the extent of inhibition slightly declined at fibrinogen levels above 4 g/L (Figure 7B). Further analysis demonstrated a strong correlation of the curve parameters with and without Nb106 (Figure 7C), thus indicating that the overall process of thrombin generation is driven by other plasma factors than fibrin(ogen).

To study the role of fibrin-bound thrombin under conditions of partial anticoagulation, we performed measurements in the presence of reversible thrombin inhibitor dabigatran, or of the protein C pathway activators, thrombomodulin and activated protein C (APC). Markedly, the Nb106 had similar inhibitory effects on the thrombin generation process in all these conditions (Figure 16). This confirmed a secondary effect of fibrin on the thrombin generation process.

Considering the role of fibrin in thrombin generation, we investigated this process in patients diagnosed with congenital dysfibrinogenemia, in the majority with a bleeding or prolonged clotting time phenotype (Table 1). Obtained were plasmas from six patients with defined *FGA* mutations, four patients with *FGB* mutations and five patients with FGG mutations. In all patients functional fibrinogen levels were low, *i.e.* in 13/15 cases below the reference range of 1.5-4.5 g/L, as determined by the Clauss method based on clotting times (Table 1). The 2 remaining patients plus 7 other patients had fibrinogen antigen levels below the reference range of 80-120%, thus pointing to an aberrant fibrinogen function or antigen level in all cases. Dot plots of the thrombin peak height or ETP as a function of fibrinogen (Clauss) did not reveal marked changes in the plasma thrombin generation when comparing patients with the healthy control group (Figure 8A). However, when thrombin generation was measured in the presence of Nb106, plots showed a marked lowering of the nanobody effect (Figure 8B), in agreement with the low fibrinogen clotting time in the patient plasmas.

### Thrombin binding determines the fibrin clot structure

Considering the major effects of Nb106-induced thrombin replacement on thrombin generation, we also examined the fibrin clots. We first measured the mechanical clotting time by plasma triggering with tissue factor (1-1000 pM) plus phospholipids. The Nb106 did not influence the kinetics, regardless of the concentration of tissue factor (Figure 9A). Apparently, the clots formed had sufficient resistance to stop the stirring ball. Strikingly, when performing a turbidity-based clotting assay, we found that the nanobody almost completely suppressed the optical density changes in tissue factor-triggered plasma (Figure 9B). Together, this suggested that Nb106 did not abolish clot formation, but changed the fibrin structure.

To study the physical properties of the clots, we then monitored the nanobody effect on tissue factor-induced clot retraction under stasis. Under control conditions, in whole blood and PRP the triggering resulted in a gradual formation of macroscopic, contracting fibrin clots over a period of 150 min (Figure 10A). Applying Nb106 delayed this process and resulted in the formation of essentially non-contracting clots (Figure 10A-C). This result implied that the thrombin replacement from fibrin greatly altered the structure and function of fibrin clots.

We used scanning electron microscopy to observe this alteration of the fibrin structure by Nb106 at higher resolution, by using plasma, PRP and whole blood samples that were preincubated with increasing concentrations Nb106 (0-100 μg/mL) and triggered with tissue factor. As shown in Figure 11, the dosing of Nb106 led to a gradual change in fibrin structure from fibrillar networks to amorphous clumps. In contrast, the irrelevant nanobody Nb-Syn1C6 did not alter the normal structure of fibrin fibres. We then used light transmission microscopy to also follow the clotting process in time. Under static control conditions with normal plasma, after 10 min of tissue factor addition, we observed typical fibrin networks, which gradually grew in density (Figure 17). In the presence of Nb106, however, visible fibrin structures formed more slowly, consisting of irregular curvilinear structures. Interestingly, when using antithrombin-deficient plasma, the fibrin networks formed similarly as in control plasma, although the fibres appeared thicker in size. Upon application of Nb106 to antithrombin-deficient plasma, the fibrin formed again had a curvilinear appearance, with structures that appeared denser, when compared to normal pool plasma. These results demonstrated that the Nb106 altered the temporal and spatial growth of fibrin formation, especially restricting fibrin fiber extension.

### Altered fibrin-thrombus formation in mouse blood under flow

As a preparation to investigate the importance of fibrin-bound thrombin in haemostasis and thrombosis *in vivo,* we studied the effects of Nb106 on thrombin generation in platelet-rich plasma from wild-type C57BL/6 mice. As shown in Figure 12A, it appeared that the nanobody suppressed the thrombin generation process similarly as in human plasma. Under stasis, it also abolished the formation of long fibrin fibres, changing these into more amorphous structures (Figure 12B). We then used an established microfluidic protocol of collagen- and tissue factor-dependent platelet-fibrin thrombus formation with perfused whole mouse blood. Upon blood flow at a low, venous wall-shear rate, we found that the application of Nb106 (100 mg/mL) greatly reduced and delayed fibrin formation, along with the formation of large platelet thrombi (Figure 12C-D). However, when judging the contraction of platelet thrombi, such as exemplified by the thrombus score, a difference at t=8 min was observed (Figure 12E). This indicated that locally still sufficient thrombin was present to allow platelet aggregate contraction. When we performed flow experiments with mouse blood at a high, arterial shear rate, Nb106 was no longer effective in reducing the formation of large thrombi and fibrin (Figure 18A-C).

Accordingly, as an interference tool, the nanobody is expected to be most effective under static and low-shear conditions, such as in venous thrombosis.

In this study, the novel nanobody Nb106 was employed, raised against fibrin degradation products, to re-investigate the complex interactions between thrombin and fibrin. The nanobody was positively selected for binding to fibrin and negatively for binding to fibrinogen. After the finding that the Nb106 was able to suppress the thrombin generation potential in normal plasma, PRP and whole blood for 40-60%, we established that it was no longer effective in the absence of fibrinogen or antithrombin or when applied after the onset of thrombin generation. Prothrombin consumption measurements then indicated a 2.2-fold increased lifetime in thrombin generation of fibrin-bound thrombin (displaced by Nb106) in comparison to the soluble pool of thrombin. Detailed examination of fibrin structures showed that the fibrin-retained thrombin was required for clot retraction and the formation of elongating fibrin fibres. Jointly these data point to the presence of a substantial pool of proteolytically active thrombin that binds to fibrin, is protected from anticoagulation and serves to further promote fibrin clot formation (Figure 13). Accordingly, fibrin rather acts as a re-director of thrombin's targets. Differently, also endothelial-expressed thrombomodulin is doing so, in terms of activating protein C.

Regarding thrombin generation assays, in 2003 the question was raised: what is all that thrombin for? We can now state that a substantial amount is required for making well-structured fibrin clots. From a different point of view, our results contribute to a better understanding of the outcome of integrative thrombin generation assays, e.g. triggered by tissue factor. A substantial part of the measured thrombin is dependent on formed fibrin (rather than on fibrinogen) and then partly protected against native anticoagulation mechanisms via antithrombin. However, we also find that anticoagulants such as dabigatran and thrombomodulin do not affect the contribution of fibrin-bound thrombin to the thrombin generation potential.

Hence, the activity of fibrin-bound thrombin is not expected to change assignments of the thrombophilia or haemophilia state, and the inclusion of the nanobody in future tests may provide additional insight into parameters regulating thrombin dynamics.

Patients with congenital dysfibrinogenemia due to mutations in the fibrinogen genes FGA, FGB or FGG, present with heterozygous symptoms, although a mild bleeding phenotype is more frequently observed than a thrombophilia phenotype. This was also the case for the 15 patients included in the present study. Plasmas from the majority of patients showed low functional fibrinogen levels, which translated in thrombin generation assays to relatively low suppressive effects of Nb106. This suggests that the extent of inhibition achieved by the nanobody can predict fibrin-dependent hemostatic activity. Our ex vivo flow studies with mouse blood furthermore showed a more profound inhibition of fibrin formation at venous shear rate than at arterial shear rate, while leaving platelet thrombus formation unchanged. Further experiments to define the suitability of Nb106 as a therapeutic tool for the downregulation of venous fibrin clots are thus important to perform.

**Table 1. Fibrinogen characteristics and thrombin generation in patients with congenital dysfibrinogenemia.** Shown are basic patient characteristics, including established fibrinogen gene mutations. Thrombin generation in plasmas was triggered with tissue factor/phospholipids in the presence of a vehicle control medium or Nb106 (100 µg/mL), as described in Figure 3. Reference values of functional fibrinogen (Clauss method) were 1.5-4.5 g/L, and reference values of fibrinogen antigen were 80-120%.

| Patient | Sex | Age | Fibrinogen variants | Phenotype | Fibrinogen (Claus) (g/L) | Fibrinogen antigen (%) | Thr peak control (nM) | Thr peak Nb106 (nM) | Thr peak inhibition (%) | ETP control (nM.min) | ETP Nb106 (nM.min) | ETP inhibition (%) |
|---|---|---|---|---|---|---|---|---|---|---|---|---|
| FGA1 | M | 28 | FGA c.95 G>A (p.G32E) het | prolonged PT | 1.06 | 88 | 678 | 382 | 43.7% | 3403 | 1570 | 53.9% |
| FGA2 | M | 62 | FGA c.95 G>A (p.G32E) het | epistaxis in childhood | 1.08 | 101 | 504 | 225 | 55.4% | 3225 | 1292 | 59.9% |
| FGA3 | M | 53 | FGA c.112 A>G (p.R38G) het | prolonged PT | 0.65 | 101 | 328 | 155 | 52.7% | 3056 | 1248 | 59.2% |
| FGA4 | F | 61 | FGA c.149 C>G (p.S50*) hom | intercranial bleeding, aortic thrombosis | <0.35 | 10 | 320 | 271 | 15.3% | 2470 | 2154 | 12.8% |
| FGA5 | F | 63 | FGA c.112 A>G (p.R38G) het / FGB c.794 C>T (p.P265L) het | family study | 1.04 | 106 | 202 | 88 | 56.4% | 1972 | 873 | 55.8% |
| FGA6 | F | 22 | FGA c.112 A>G (p.R38G) het | prior bleeding | 0.58 | 80 | 297 | 145 | 51.2% | 2613 | 1293 | 50.5% |
| FGB1 | M | 5 | FGB c.886 T>C (p.W296R) hom | prior bleeding | 0.68 | 37 | 329 | 239 | 27.4% | 2233 | 1633 | 26.9% |
| FGB2 | M | 38 | FGB c.886 T>C (p.W296R) het | bleeding after minor injuries | 1.06 | 46 | 531 | 249 | 53.1% | 3088 | 1243 | 59.7% |
| FGB3 | F | 26 | FGB c.886 T>C (p.W296R) het | family study | 1.09 | 57 | 559 | 336 | 39.9% | 3971 | 2631 | 33.7% |
| FGB4 | M | 28 | FGB c.534 G>C (p.K178N) het | none | 1.61 | 66 | 141 | 67 | 52.5% | 1473 | 701 | 52.4% |
| FGG1 | M | 44 | FGG c.998 A>G (p.H333R) het | thrombosis of superior mesenteric vein | 0.42 | 28 | 386 | 273 | 29.3% | 2680 | 1776 | 33.7% |
| FGG2 | F | 13 | FGG c.998 A>G (p.H333R) het | prior bleeding | 0.91 | 38 | 271 | 148 | 45.4% | 2059 | 1161 | 43.6% |
| FGG3 | M | 24 | FGG c.1223 C>T (p.T408I) het | prolonged PT | 0.85 | 29 | 276 | 155 | 43.8% | 2199 | 1223 | 44.4% |
| FGG4 | M | 41 | FGG c.323 C>G (p.A108G) het | prior bleeding | 1.60 | 58 | 114 | 58 | 49.1% | 1117 | 594 | 46.8% |
| FGG5 | F | 27 | FGG c.1007 T>C (p.M336T) het | menorrhagia, prolonged PT | 0.35 | 142 | 99 | 69 | 30.3% | 797 | 804 | -0.9% |

## Example 2

The fibrinolytic system is crucial for clot lysis during wound healing. Clot lysis is initiated by tissue plasminogen activator (tPA) and urokinase plasminogen activator (uPA), which convert plasminogen into plasmin. TPA-mediated plasminogen activation requires binding to fibrin, while uPA activates plasminogen independent of fibrin. Both plasminogen activators are inhibited by plasminogen activator inhibitor-1 (PAI-1), although, fibrin-bound tPA is protected from PAI-1. As inadequate regulation of fibrinolysis might lead to thrombotic and bleeding events, there is a clinical need for tools that estimate a patient's fibrinolytic state. We developed anti-fibrin-uPA (aFn-uPA), a construct containing the plasminogen activator uPA, linked to a nanobody directed against fibrin, to support the binding of uPA to fibrin, and to increase the effectiveness of plasminogen activation. This novel plasminogen activator improves the sensitivity of the calibrated, automated plasmin generation (PG) assay, for PAI-1.

The aim is to increase the sensitivity of the calibrated, automated PG assay for the detection of (anti-)PAI-1 effects in human plasma.

## Methods

PG in normal pooled plasma (NPP) or factor-deficient plasma was measured by cleavage of a plasmin-specific fluorogenic substrate, using a calibrated, automated method. In brief, fibrinolysis was started by combining plasma and trigger solution in a 96-well plate. The trigger solution consists of tissue factor (TF) or Russell's viper venom-factor X activator (RVV-X), phospholipids, and tPA or aFn-uPA. PG was measured with a fluorometer, equipped with a 390/460 filter set (excitation/emission) (Fluoroscan Ascent, Thrombinoscope, Maastricht, The Netherlands). The following PG parameters were extracted from the data: lag time, time to peak (ttPeak), endogenous plasmin potential (EPP) and peak.

## Results

High TF, RVV-X, tPA and aFn-uPA were associated with a shorter PG lag time and a shorter PG ttPeak, compared to lower trigger concentrations. For subsequent experiments, 5 pM TF or 10 -5 RVV-X, and 1.25 $\mu$g/mL (17.8 nM) tPA or 1 $\mu$g/mL (21 nM) aFn-uPA, were selected as optimal. The assay was specific for plasmin as no PG was observed in plasminogen- nor fibrinogen-deficient plasma. The sensitivity of the PG assay to PAI-1 was higher in PG triggered with the novel plasminogen activator aFn-uPA, compared to tPA, regardless of the affinity of both activators for fibrin. PAI-1 dose-dependently increased the lag time and ttPeak, and reduced the EPP and peak in PG stimulated with aFn-uPA in both NPP and PAI-1-deficient plasma. Using an anti-PAI-1-Ab, the inhibition obtained upon 2 $\mu$g/mL (46.5 nM) PAI-1, was reduced or completely abolished, depending on the concentration of anti-PAI-1-Ab (Figure 19).

Overall, the calibrated, automated PG assay, in particular when aFn-uPa is used as a trigger for fibrinolysis, is a specific and efficient tool to measure PG in human plasma and is sensitive to the therapeutic target PAI-1. This tool may help identify abnormal fibrinolytic states in bleeding and thrombotic disorders.

## Example 3

Thrombin has a multitude of roles in the initiation and propagation of (anti)coagulation, in platelet activation and fibrin clot formation. The assessment of thrombin generation (TG) and thrombin-dynamics, combined with multi-omics data, has provided in-depth understanding of the plasma factors contributing to the (in)activation of thrombin in triggered plasma and whole-blood. By default, the generated thrombin is considered to act as a single pool. Evidence is presented for at least two major proteolytically active thrombin pools with non-overlapping functions in blood clotting.

## Methods

A novel Syn-Nb-AF106 anti-fibrin nanobody (Nb106) was characterized, targeting a species-conserved thrombin-binding site exposed by fibrinopeptide A cleavage from fibrinogen. Thrombin generation was assessed by calibrated automated thrombography using various triggers with control (multi-donor) and (reconstituted) coagulation factor-deficient plasmas, platelet-rich plasma or whole blood. Fibrin clot formation was evaluated by scanning electron microscopy (SEM). Clot retraction was macroscopically assessed.

## Results

In tissue factor-triggered thrombin generation, Nb106 dose-dependently reduced the thrombin peak level and endogenous thrombin potential (ETP) to 54-62%, without affecting kinetic thrombin generation parameters. A similar reduction in platelet-free plasma, platelet-rich plasma and whole blood. Biochemically, Nb106 displaced thrombin from a binding site on cleaved fibrinogen. Nb106 inhibited tissue factor-induced thrombin generation, using plasma deficient in factor IX, XI or XII, but not using plasmas deficient in fibrinogen or antithrombin. Reconstitution experiments showed that the inhibition depended on the fibrinogen concentration. A-specific control nanobodies were without effect. Together, the fibrin(ogen)-dependent and thrombin-inhibiting effect indicated that Nb106 operates by releasing proteolytically-active thrombin from fibrin to allow inactivation by antithrombin.

Time-dependent, late spiking experiments revealed that Nb106 reduced the generation of thrombin only when added at 0-15 minutes after trigger, but no longer after 30 minutes, suggesting that the inhibitory capacity was due to a shortened thrombin binding to fibrin rather than to a continued protection towards antithrombin. In plasmas from a cohort of 64 healthy subjects, application of Nb106 showed a consistent reducing effect on the parameters peak and ETP, with a moderate positive correlation with the fibrinogen level. Nb106 suppressed the thrombin generation amplitude, but not the kinetics in the presence of a panel of direct oral anticoagulants (DOAC). Strikingly, brightfield microscopy and SEM indicated that Nb106, but not control nanobodies, in triggered plasma, platelet-rich plasma and whole-blood, fully abrogated the formation of elongating fibrin fibers. In agreement with this, it strongly suppressed whole-blood clot retraction.

Collectivity, these data point to the existence of two major pools of proteolytically active thrombin, both of which contributing to thrombin generation, that are formed upon plasma and blood coagulation. One pool of soluble thrombin steers the proteolytic coagulation cascade, while another pool is bound to growing fibrin fibers. The latter pool still cleaves the conventional AMC thrombin substrate, is temporarily protected against antithrombin, and is required for fibrin fiber extension and blood clotting and clot retraction. The Nb106, replacing thrombin from its binding site on fibrin, selectively annuls the second pool, thereby blocking the clotting process. The replacement ensures thrombin inactivation by antithrombin.

**Example 4**

**Methods**

Coagulation was induced in autologous whole blood and PRP by recalcification (10 mM $CaCl_2$) and 1 pM tissue factor. Samples contained vehicle medium (control), a VHH antibody according to an embodiment of the invention (o-FN) and a polypeptide according to the invention comprising VHH and uPA (o-FN-uPA). Formation and retraction of fibrin clot was imaged after 0, 20, 40, 80, 150 and 210 min.

**Results**

To study the physical properties of the clots, the effect of the VHH as well as the WHH-uPA, according to embodiments of the invention, on tissue factor-induced clot retraction under stasis, were monitored. Under control conditions, in whole blood and PRP the triggering resulted in a gradual formation of macroscopic, contracting fibrin clots over a period of 210 min (Figure 22). Applying the VHH delayed this process and resulted in the formation of essentially non-contracting clots while the VHH-uPA polypeptide prevented the clot formation. This result underlines the potency of VHH-uPA in treating thrombotic events.

**SEQUENCES**

SEQ ID N°1 (CDR1 of VHH1) GTSFDINT

SEQ ID N°2 (CDR1 of VHH2) GTILDINT

SEQ ID N°3 (CDR1 of VHH3) GSIFSINA

SEQ ID N°4 (CDR1 of VHH4) GRTFSTAT

SEQ ID N°5 (CDR2 of VHH1) NTEGITN

SEQ ID N°6 (CDR2 of VHH2) TSAGITD

SEQ ID N°7 (CDR2 of VHH3) TSGGSTN

SEQ ID N°8 (CDR2 of VHH4) TVTGGVTRSAL

SEQ ID N°9 (CDR3 of VHH1) NAWIRGINRLYDNY

SEQ ID N°10 (CDR3 of VHH2) NAWVRGTNRLYDNY

SEQ ID N°11 (CDR3 of VHH3) NVNYYNDYGVPYDY

SEQ ID N°12 (CDR3 of VHH4) AAVGRLGVFARANEYDY

SEQ ID N°13 (FR1 of VHH1 to VHH3) EVQLVESGGGLVQPGGSLRLSCAAS

SEQ ID N°14 (FR1 of VHH4) EVQLVESGGGLVQAGGSLRLSCAAS

SEQ ID N°15 (FR2 of VHH1) MTTWYRQAPGAQRELVATI

SEQ ID N°16 (FR2 of VHH2) MTWYRQPGAQRELVATI

SEQ ID N°17 (FR2 of VHH3) MGWYRQAPGKQRELVAAI

SEQ ID N°18 (FR2 of VHH4) MGWFRQAPGKEREFVARI

SEQ ID N°19 (FR3 of VHH1) YADFVEGRFTISRDNAKNTVYLQMNNLKPEDTAVYYC

SEQ ID N°20 (FR3 OF VHH2) YADFVKGRFAISRDNTKNTVYLQMNNLKPEDTAVYYC

SEQ ID N°21 (FR3 of VHH3) YADSVKGRFTISRDNAKSTVYLQMYSLKPEDTAVYFC

SEQ ID N°22 (FR3 of VHH4) YGDPVKGRFTISRDNAKNTVYLQMANLKPEDTAVYYC

SEQ ID N°23 (FR4 OF VHH1, VHH2 AND VHH4) WGQGTQVTVSS

SEQ ID N°24 (FR4 OF VHH3) WGQGTRVTVSS

SEQ ID N°25 (VVH1)

EVQLVESGGGLVQPGGSLRLSCAASGTSFDINTMTTWYRQAPGAQRELVATINTEGITNYAD
FVEGRFTISRDNAKNTVYLQMNNLKPEDTAVYYCNAWIRGINRLYDNYWGQGTQVTVSS

SEQ ID N°26 (VHH2)

EVQLVESGGGLVQPGGSLRLSCAASGTILDINTMTWYRQPGAQRELVATITSAGITDYADFV
KGRFAISRDNTKNTVYLQMNNLKPEDTAVYYCNAWVRGTNRLYDNYWGQGTQVTVSS

SEQ ID N°27 (VHH3)

EVQLVESGGGLVQPGGSLRLSCAASGSIFSINAMGWYRQAPGKQRELVAAITSGGSTNYAD
SVKGRFTISRDNAKSTVYLQMYSLKPEDTAVYFCNVNYYNDYGVPYDYWGQGTRVTVSS

SEQ ID N°28 (VHH4)

EVQLVESGGGLVQAGGSLRLSCAASGRTFSTATMGWFRQAPGKEREFVARITVTGGVTRSA
LYGDPVKGRFTISRDNAKNTVYLQMANLKPEDTAVYYCAAVGRLGVFARANEYDYWGQGTQ
VTVSS

SEQ ID N°29 (uPA)

KPSSPPEELKFQCGQKTLRPRFKIIGGEFTTIENQPWFAAIYRRHRGGSVTYVCGGSLMSPCW
VISATHCFIDYPKKEDYIVYLGRSRLNSNTQGEMKFEVENLILHKDYSADTLAHHNDIALLKIR
SKEGRCAQPSRTIQTICLPSMYNDPQFGTSCEITGFGKENSTDYLYPEQLKMTVVKLISHREC
QQPHYYGSEVTTKMLCAADPQWKTDSCQGDSGGPLVCSLQGRMTLTGIVSWGRGCALKDK
PGVYTRVSHFLPWIRSHTKEENGLAL

SEQ ID N°30 (linker) SSSGSG

SEQ ID N°31 (consensus sequence)

EVQLVESGGGLVQX1GGSLRLSCAASGX2X3XXXXXMXXWXRXXPGXXREXVAXIXXXGXX
XXXXXXYXDXVXGRFXISRDNXKXTVYLQMXXLKPEDTAVYXCXXXXXXXXXXXXXXXXXXYWG
QGTXVTVSS

SEQ ID N°32 (region between the 17$^{th}$ and 50$^{th}$ amino acids from the N-terminal end of the fibrin $\alpha$-chain)
GPRVVERHQSACKDSDWPFCSDEDWNYKCPSGCR

SEQ ID N°33 (region between the 33$^{rd}$ and 39$^{th}$ amino acids from the N-terminal end of the fibrin $\alpha$-chain) WPFCSDE

SEQ ID N°34 (fibrin $\alpha$-chain)

ADSGEGDFLAEGGGVRGPRVVERHQSACKDSDWPFCSDEDWNYKCPSGCRMKGLIDEVN
QDFTNRINKLKNSLFEYQKNNKDSHSLTTNIMEILRGDFSSANNRDNTYNRVSEDLRSRIEV
LKRKVIEKVQHIQLLQKNVRAQLVDMKRLEVDIDIKIRSCRGSCSRALAREVDLKDYEDQQK
QLEQVIAKDLLPSRDRQHLPLIKMKPVPDLVPGNFKSQLQKVPPEWKALTDMPQMRMELERP
GGNEITRGGSTSYGTGSETESPRNPSSAGSWNSGSSGPGSTGNRNPGSSGTGGTATWKPG
SSGPGSTGSWNSGSSGTGSTGNQNPGSPRPGSTGTWNPGSSERGSAGHWTSESSVSGST
GQWHSESGSFRPDSPGSGNARPNNPDWGTFEEVSGNVSPGTRREYHTEKLVTSKGDKELR
TGKEKVTSGSTTTTRRSCSKTVTKTVIGPDGHKEVTKEVVTSEDGSDCPEAMDLGTLSGIGT
LDGFRHRHPDEAAFFDTASTGKTFPGFFSPMLGEFVSETESRGSESGIFTNTKESSSHHPGIA
EFPSRGKSSSYSKQFTSSTSYNRGDSTFESKSYKMADEAGSEADHEGTHSTKRGHAKSRPV
RDCDDVLQTHPSGTQSGIFNIKLPGSSKIFSVYCDQETSLGGWLLIQQRMDGSLNFNRTWQ
DYKRGFGSLNDEGEGEFWLGNDYLHLLTQRGSVLRVELEDWAGNEAYAEYHFRVGSEAEGY
ALQVSSYEGTAGDALIEGSVEEGAEYTSHNNMQFSTFDRDADQWEENCAEVYGGGWWYNN
CQAANLNGIYYPGGSYDPRNNSPYEIENGVVWVSFRGADYSLRAVRMKIRPLVTQ

SEQ ID N°35 (thrombin exosite-1 binding site on fibrin) WPFCSDE

SEQ ID N°36 (fibrin α-chain 60 amino acids from the N teminus)

ADSGEGDFLAEGGGVRGPRVVERHQSACKDSDWPFCSDEDWNYKCPSGCRMKGLIDEVN

Q

SEQ ID N°37 (linker) GGGGSGGGGSGGGGS

SEQ ID N°38 (linker) EAAAKEAAAKEAAAK

SEQ ID N°39 (linker) PAPAP

**Claims**

1.  A polypeptide comprising an immunoglobulin single variable domain and an urokinase-type plasminogen activator (uPA) domain wherein the immunoglobulin single variable domain is able to bind to fibrin via a fibrin-binding domain.

2.  The polypeptide of claim 1, wherein said fibrin-binding domain is comprised in a single variable domain on a heavy chain (VHH) domain or a fragment thereof.

3.  The polypeptide, according to claim 2, wherein the VHH specifically binds to the fibrin region comprised between the 17$^{th}$ and 50$^{th}$ amino acids from the N-terminal end of the fibrin α-chain according to SEQ ID N°32, more preferably between the 33$^{rd}$ and 39$^{th}$ amino acids from the N-terminal end of the fibrin α-chain according to SEQ ID N°33.

4.  The polypeptide according to any of the claims 2 or 3, wherein the VHH binds to human fibrin with an affinity 1000 times higher than to human fibrinogen.

5.  The polypeptide according to any of the claims 1 to 4, wherein the polypeptide exhibits fibrinolytic activity in plasma or whole blood.

6.  The polypeptide according to claim 5, wherein said fibrinolytic activity is 1000 times higher than the fibrinolytic activity of uPAs.

7.  The polypeptide according to any of the claims 1 to 6, wherein the polypeptide exhibits thrombolytic activity.

8.  The polypeptide, according to any of the claims 2 to 7, wherein the VHH has an amino acid sequence according to SEQ ID N°25, SEQ ID N°26, SEQ ID N°27, or SEQ ID N°28.

9.  The polypeptide according to any of the previous claims, wherein the uPA has an amino acid sequence according to SEQ ID N°29.

10. The polypeptide according to any of the claims 2 to 9, wherein the C terminus of the VHH and the N terminus of the uPA domain are linked through a linker.

11. A composition comprising a peptide according to any of the claims 1 to 10 and a pharmaceutical-acceptable carrier, excipient or diluent.

12. The composition according to claim 11, wherein said composition is formulated for parenteral administration.

13. A peptide according to any of the claims 1 to 10 or a composition according to any of the claims 11 to 12, for use in the treatment, prevention or amelioration of a disease or condition **characterized by** a thrombotic or embolic state in a subject in need thereof.

14. The peptide or composition for use according to claim 13 wherein the disease or condition **characterized by** a thrombotic or embolic state is selected from embolism, lung embolism, stroke, infarction, brain infarction, thrombotic stroke, a microthrombotic disorder, venous occlusive disease, ischemia, thrombosis in dialysis patients, acute myocardial infarction, deep vein thrombosis, acute ischemic stroke, acute peripheral arterial occlusion, occlusion of indwelling catheters, intracardiac thrombus formation and microthrombotic ischemia.

15. The peptide or composition for use according to any of the claims 13 or 14, in emergency medicine, or wherein the disease or condition is an acute or chronic disease or condition.

16. A peptide according to any of the claims 1 to 10 or a composition according to any of the claims 11 to 12, for use in the diagnosis of abnormal fibrinolytic states in bleeding and thrombotic disorders.

FIG. 1

**FIG. 2**

**FIG. 3**

**FIG. 4**

**FIG. 5**

**FIG. 6**

FIG. 7

A

B

**FIG. 8**

A

B (i)

(ii)

**FIG. 9**

**FIG. 10**

**FIG. 11**

**FIG. 12**

**FIG. 13**

**FIG. 14**

| PRP deficient in | Control % | Nb106 effect (% of control) | | | | | |
|---|---|---|---|---|---|---|---|
| | | Lagtime (min) | P value | Peak (nM) | P value | ETP (nM x min) | P value |
| None | 100 | 98.25 ± 2.48 | 0.3739 | 56.38 ± 6.60 | 0.0011 | 59.86 ± 1.01 | 0.0001 |
| Fibrinogen | 100 | 99.76 ± 0.33 | 0.4226 | 99.76 ± 0.33 | 0.1701 | 97.60 ± 1.34 | 0.1371 |
| Factor IX | 100 | 101.59 ± 2.24 | 0.5185 | 69.46 ± 2.82 | <0.0001 | 70.27 ± 2.28 | 0.0001 |
| Factor XI | 100 | 97.62 ± 3.37 | 0.3739 | 68.14 ± 2.23 | <0.0001 | 69.20 ± 1.18 | <0.0001 |
| Factor XII | 100 | 100.68 ± 0.96 | 0.3739 | 57.30 ± 2.72 | <0.0001 | 71.62 ± 3.25 | 0.0001 |
| Antithrombin | 100 | 95.83 ± 2.95 | 0.1161 | 95.13 ± 0.40 | 0.0832 | 100.23 ± 1.47 | 0.8135 |

**FIG. 15**

**FIG. 16**

**FIG. 17**

**FIG. 18**

**FIG. 19**

CLUSTAL O(1.2.4) multiple sequence alignment

```
SEQ_ID31_consensus    EVQLVESGGGLVQXGGSLRLSCAASGXXXXXXXMXXWXRXXPGXXREXVAXIXXXGXXXX    60
SEQ_ID28_VHH4         EVQLVESGGGLVQAGGSLRLSCAASGRTFSTATMG-WFRQAPGKEREFVARITVTGGVTR    59
SEQ_ID27_VHH3         EVQLVESGGGLVQPGGSLRLSCAASGSIFSINAMG-WYRQAPGKQRELVAAITSGGS---    56
SEQ_ID25_VVH1         EVQLVESGGGLVQPGGSLRLSCAASGTSFDINTMTTWYRQAPGAQRELVATINTEGI---    57
SEQ_ID26_VHH2         EVQLVESGGGLVQPGGSLRLSCAASGTILDINTMT-WYR-QPGAQRELVATITSAGI---    55
                      **************  *************       *  *   **  ** **  *    *

SEQ_ID31_consensus    XXXYXDXVXGRFXISRDNXKXTVYLQMXXLKPEDTAVYXCXXXXXXXXXXXXXXXXXYWGQ    120
SEQ_ID28_VHH4         SALYGDPVKGRFTISRDNAKNTVYLQMANLKPEDTAVYYCAAVGRLGVFARANEYDYWGQ    119
SEQ_ID27_VHH3         -TNYADSVKGRFTISRDNAKSTVYLQMYSLKPEDTAVYFCNVNYYN---DYGVPYDYWGQ    112
SEQ_ID25_VVH1         -TNYADFVEGRFTISRDNAKNTVYLQMNNLKPEDTAVYYCNAWIRG---INRLYDNYWGQ    113
SEQ_ID26_VHH2         -TDYADFVKGRFAISRDNTKNTVYLQMNNLKPEDTAVYYCNAWVRG---TNRLYDNYWGQ    111
                       *  *  *** ***** *  ******   *********  *                ****

SEQ_ID31_consensus    GTXVTVSS         128
SEQ_ID28_VHH4         GTQVTVSS         127
SEQ_ID27_VHH3         GTRVTVSS         120
SEQ_ID25_VVH1         GTQVTVSS         121
SEQ_ID26_VHH2         GTQVTVSS         119
                      **  *****
```

**FIG. 20**

CLUSTAL O(1.2.4) multiple sequence alignment

```
SEQ_ID34_fibrin_a_chain    ADSGEGDFLAEGGGVRGPRVVERHQSACKDSDWPFCSDEDWNYKCPSGCRMKGLIDEVNQ    60
SEQ_ID32_AA17_to_AA50      ----------------GPRVVERHQSACKDSDWPFCSDEDWNYKCPSGCR----------    34
SEQ_ID_33_AA33_to_AA39     ------------------------------WPFCSDE---------------------    7
                                                         *******
```

**FIG. 21**

**FIG. 22**

Europäisches
Patentamt

European
Patent Office

Office européen
des brevets

## EUROPEAN SEARCH REPORT

**Application Number**

EP 24 17 3920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE SIMONE ILARIA ET AL: "A Novel Plasminogen Activator Anti-Fibrin-uPA Increases the Sensitivity of the Plasmin Generation Assay to Therapeutic Target PAI-1", BLOOD, vol. 142, no. Supplement 1, 2 November 2023 (2023-11-02), pages 5464-5464, XP093216860, US ISSN: 0006-4971, DOI: 10.1182/blood-2023-181509 * "Introduction" * * "Conclusion" * | 1,2,4-7, 11,16 | INV. C07K16/18 A61P7/02 |
| X | Hagemeyer C E ET AL: "Construction and characterization of a recombinant plasminogen activator composed of an anti-fibrin single-chain antibody and low-molecular-weight urokinase", Journal of Thrombosis and Haemostasis, 1 January 2004 (2004-01-01), pages 797-803, XP093216878, Retrieved from the Internet: URL:https://pdf.sciencedirectassets.com/78 2959/1-s2.0-S1538783604X25009/1-s2.0-S1538 783622157739/main.pdf?X-Amz-Security-Token =IQoJb3JpZ2luX2VjECoaCXVzLWVhc3QtMSJIMEYCI QCv2SIyzexMOaS2KHc80Nz2W0WUqcTt74TWKUW4iQC R3gIhAN36%2FV4HAyJ8PGv6VSu%2B80h6vSpLNXjxt 68LhnPytU11KrwFCJL%2F%2F%2F%2F%2F%2F%2F%2F %2F%2FwEQB * abstract * -----  -/-- | 1-16 | TECHNICAL FIELDS SEARCHED (IPC) C07K A61P |

The present search report has been drawn up for all claims

| Place of search | Date of completion of the search | Examiner |
|---|---|---|
| Munich | 22 October 2024 | Malamoussi, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

**Application Number**

**EP 24 17 3920**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 355 068 A2 (GEN HOSPITAL CORP [US]) 21 February 1990 (1990-02-21) * column 16, line 55 - column 18, line 34 * * claim 6 * ----- | 1-16 | |
| X | US 5 609 869 A (QUERTERMOUS THOMAS [US] ET AL) 11 March 1997 (1997-03-11) * example 5 * ----- | 1-16 | |

TECHNICAL FIELDS
SEARCHED        (IPC)

The present search report has been drawn up for all claims

| Place of search | Date of completion of the search | Examiner |
|---|---|---|
| Munich | 22 October 2024 | Malamoussi, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 644 415 A1**

## ANNEX TO THE EUROPEAN SEARCH REPORT
## ON EUROPEAN PATENT APPLICATION NO.

EP 24 17 3920

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2024

| Patent document cited in search report | | Publication date | Patent family member(s) | | Publication date |
|---|---|---|---|---|---|
| EP 0355068 | A2 | 21-02-1990 | CA | 1339546 C | 18-11-1997 |
| | | | EP | 0355068 A2 | 21-02-1990 |
| | | | WO | 9002338 A1 | 08-03-1990 |
| US 5609869 | A | 11-03-1997 | NONE | | |

EPO FORM P0459

For more details about this annex : see Official Journal of the European Patent Office, No. 12/82